# EUROPEAN PATENT SPECIFICATION

(11) **EP 0 910 294 B1**
(45) Date of publication and mention of the grant of the patent: **03.01.2007**
(21) Application number: 97931520.7
(22) Date of filing: 30.06.1997
(51) Int. Cl.: A61B 17/32, A61B 17/28

(54) **FINGERTIP-MOUNTED MINIMALLY INVASIVE SURGICAL INSTRUMENTS**
AN FINGERSPITZEN ANGEBRACHTES INSTRUMENT ZUR MINIMAL-INVASIVEN CHIRURGIE
INSTRUMENTS CHIRURGICAUX MINIMALEMENT INVASIFS MONTES SUR L'EXTREMITE DES DOIGTS

(30) Priority: 01.07.1996 US 17854 P; 04.03.1997 US 811546
(43) Date of publication of application: 28.04.1999
(60) Divisional application: 06076829.8
(73) Proprietor: University of Massachusetts, Boston, MA 02108 (US); SMITH & NEPHEW DYONICS INC, Andover, Massachusetts 01810 (US)
(72) Inventor: EK, Steven, Bolton, MA 01740 (US); BEANE, Richard, M., Hingham, MA 02043 (US); MEYERS, William, C., Worcester, MA 01609 (US)
(74) Representative: Hitchcock, Esmond Antony
(86) International application number: PCT/US1997/011494
(87) International publication number: WO 1998/000069

(56) References cited:
- EP-A- 0 070 459
- US-A- 2 668 536
- US-A- 2 704 889
- US-A- 2 781 760
- US-A- 2 811 969
- US-A- 4 726 371
- US-A- 5 242 440
- US-A- 5 383 875
- US-A- 5 423 795
- US-A- 5 450 293

## Description

This invention relates to minimally invasive surgical instruments. More particularly it relates to a finger mount having an attachment mechanism to which such an instrument may be attached.

Minimally invasive surgical techniques, including endoscopic (gastrointestinal) and laparoscopic (abdominal) procedures, employ surgical instruments that are inserted into the body through a pre-existing orifice or a small puncture or incision rather than the larger incision used in traditional, "open" surgery. Minimally invasive procedures have several advantages over open surgery, the main one being minimization of trauma to healthy tissue. As a result, recovery is accelerated and the risk of complications from infection and scar adhesion is reduced. These considerations have motivated the application of minimally invasive techniques wherever feasible. However, the instruments used in these minimally invasive procedures impair or reduce surgical access, dexterity, efficiency, and in some cases safety, when compared to the use of standard instruments in open surgery.

Nearly all minimally invasive procedures employ means for imaging the surgical site in real time. These may be non-invasive, e.g., fluoroscopy, or invasive, using, for example, an optical fiberscope. Such "scopes" can be flexible, like the endoscope, which is employed in the gastrointestinal tract, or, when the operative site is sufficiently accessible, rigid, like the laparoscope, which is used in abdominal surgery. In both endoscopy and laparoscopy, viewing light is delivered to the surgical site by fiber optics, and the surgeon views the site on an external CRT.

Laparoscopic surgery takes place in an approximately 20 x 20 x 20 cm workspace inside the patient created by insufflating the abdominal cavity with air or a gas such as carbon dioxide. The laparoscope and laparoscopic instruments are inserted into the body through a 5 to 12 mm diameter cannulae inserted through one or more puncture incisions in the abdominal wall. There are many instruments available for use in laparoscopic procedures including biopsy forceps, various types of graspers, scissors, electrocautery devices, staplers, clip appliers, needle holders, and suture loops for ligation.

In spite of the benefits, there are several limitations of the laparoscopic instruments that make laparoscopy more awkward for the surgeon than traditional, open surgery, and the nature of the instruments require a long learning curve for a surgeon to become proficient in their use. Even after learning how to use these instruments properly, surgeons still lack a certain amount of dexterity, which makes some tasks, such as suturing and knot-tying inside the body cavity, difficult.

Based on the known disadvantages, attempts have been made to improve the position, tactile, and force senses perceived by the surgeon using these laparoscopic instruments in minimally invasive procedures. Force feedback assists in suture and knot tensioning and protects against inadvertent laceration of tissue outside of the field of view of the scope. Tactile sensing is useful for manipulating suture material or other objects held with the instruments, localizing small anatomical features such as subsurface blood vessels, and detecting features that are obscured from the video camera.

Efforts at implementing tactile feedback with these instruments have focused on elaborate linkage designs or the use of complex strain sensor arrays on the tip of the instrument coupled to stimulator arrays worn on the surgeon's fingertips, e.g., on a glove, at a point remote from the tip of the instrument. A particular handmounted instrument for operating on mitral valves is disclosed in US Patent No: 2,668,536. Such systems have had some experimental success, but are complicated, both to design and to manufacture.

The present invention is directed at a finger mount for use by a surgeon comprising a finger contacting component having an inner and an outer surface with the inner surface contoured and dimensioned to fit onto a finger, and an attachment mechanism connected to the outer surface of the finger contacting component. According to the invention the attachment mechanism is rotatable relative to the finger contacting component, and configured to connect to an interchangeable miniature surgical instrument, and to enable the surgeon to actuate or deploy the instrument by use of another digit to effect rotation of the surgical instrument with respect to the finger contacting component to actuate/deploy the surgical instrument. Typically, the mount is a harness or a finger-sleeve, for securing the instrument to a fingertip.

Using the present invention a tiny surgical instrument can be mounted directly on a surgeon's fingertips in a way that the surgeon can insert his or hand into the patient through a minimal incision to perform surgical procedures, and also to use his or her fingers to manipulate tissues. This enables the surgeon to perform procedures with all the benefits of minimally invasive surgery, but with much greater tactile sense, control, and ease of manipulation, than enabled by known minimally invasive surgical instruments.

In some embodiments of the invention a retracting/deploying mechanism is operably connected to the mount; and a tool is connected directly to the retracting/deploying mechanism, or is inserted into or connected to the finger sleeve, the mechanism can then be arranged to permit the tool to be moved into a retracted position relative to the harness to expose the fingertip, and into a deployed position relative to the mount, e.g., harness, for use of the tool. The instrument can further include an attachment mechanism that rotatably connects the harness to the retracting/deploying mechanism.

The attachment mechanism can include an attachment stud and a compression member arranged to secure the retracting/deploying mechanism to the harness and to enable the retracting/deploying mechanism to rotate from a deployed position to a retracted position. The harness can include a convex outer surface that mates with a concave surface of the retracting/deploying mechanism.

In one embodiment, the tool includes a stationary jaw connected to the retracting/deploying mechanism, and a moving jaw rigidly fixed to an actuator. For example, the tool can be a grasper, a needle holder, scissors, a scalpel, or a clip applier. In a preferred embodiment, the actuator has a curved cross-section, and is curved along its longitudinal axis.

The instrument can further include a spring arranged to bias the moving jaw in an open position with respect to the stationary jaw. In addition, a portion of the retracting/deploying mechanism can be hollowed out to house the actuator when the tool is in a closed position.

In another embodiment, the mount, e.g., harness, can include openings for insertion of a strap to secure the harness to a finger. In other embodiments, the mount can be a cylindrical finger-sleeve, or can be made of a dual- or single-"wing" design made of cloth, plastic, or rubber, that is wrapped around the finger to form a cylinder, and is secured with a mechanical lock, tape, e.g., surgical tape, or a strap of hook and loop material that can be used to secure the sleeve to a finger. These tapes or straps can be fixed to the mount by welding, lamination, or glue. In addition, the wings can be secured by malleable wires embedded within, or adhered to a surface of, the wings.

In the use of a finger mount of the invention in a minimally invasive surgical procedure, a surgical instrument on the mount is secured to a fingertip of the surgeon's hand. The hand including the instrument is inserted into the patient; and the surgical procedure performed using the instrument. The method can further include securing an additional instrument onto another of the fingers prior to inserting the hand into the patient.

In variation, the instrument is secured to the finger by first attaching the mount and instrument to the finger of a glove, and then placing the glove onto the hand.

In use of the finger mount, the surgical instrument can be moved to its deployed position prior to performing the surgical procedure, and into a retracted position after performing the surgical procedure, without removing the hand from within the patient. For example, the instrument may be moved to its retracted position to enable the finger to be used to manipulate tissue in the patient, without removing the hand from within the patient.

The finger mount of the invention can include a finger contacting component having an inner and an outer surface, e.g., in the form of a semi-circle that contacts only a portion of a finger, or a cylinder that encircles the finger in use, wherein the inner surface is contoured and dimensioned to fit onto a finger, such as an index finger or thumb, and an attachment mechanism connected to the outer surface of the finger contacting component, wherein the attachment mechanism is configured to connect to a miniature surgical instrument.

In this finger mount, the attachment mechanism can be a protruding member that connects to a corresponding recess in the miniature surgical tool, or a recess that connects to a corresponding protruding member in the miniature surgical tool.

In another embodiment, the finger contacting component can include one or more straps to secure the component to the finger. These straps can each include a malleable wire that can be wrapped around the finger to secure the component, or they can be one or more wings, secured to themselves or each other by mechanical snaps or an adhesive. Further, the finger contacting component can secured to the finger by an adhesive on the inner surface of the component.

A miniature surgical instrument, eg, grasper, scissors, or needle holder, for use with a finger mount of the invention may include a rigid, elongate component having distal and proximal ends, a connector located at the proximal end of the elongate component, wherein the connector is configured to rotatably connect the instrument to the attachment mechanism of the finger mount, and a tool located at the distal end of the elongate component. For example, the tool can be a scalpel, and the elongate component can house a sliding actuator to move the scalpel from a deployed to a retracted position within the elongate component. These instruments can be used, for example, in conjunction with thumb mounts.

The mounts of the invention can also be used with a miniature surgical instrument including a rigid, elongate component having distal and proximal ends, a recess formed in the elongate component, wherein the recess is configured to connect the instrument to a protruding member in the finger mount, and a tool located at the distal end of the elongate component. Alternatively, the instrument can include a rigid, elongate component having distal and proximal ends, a protruding member attached to the elongate component, wherein the protruding member is configured to connect the instrument to a recess in the finger mount, and a tool located at the distal end of the elongate component.

The present invention can be used in minimally invasive surgical systems that include the finger mount, and a miniature surgical tool, such as a grasper, a scalpel, a needle holder, a scissor, a camera, or an electrocautery probe, that includes a connector configured to connect to the attachment mechanism of the finger mount. In certain embodiments, the miniature surgical tool can be permanently attached to the finger mount.

Finger mounts of the invention can be used with a miniature camera sealed within a housing, with a connector fixed to the housing to allow the housing to be attached to the attachment mechanism of the finger mount. In another embodiment, the instrument can be fitted with fluid aspiration or irrigation conduits, or with electrical wiring, in which case the instrument can be used to perform electrocautery of tissue in the patient.

The invention provides several advantages. One important advantage of the invention is that it enables the surgeon to perform hand-assisted minimally invasive surgery which provides the surgeon with tactile feedback that is lost when using known minimally invasive laparoscopic or endoscopic surgical instruments. standard minimally invasive surgery instruments have one or more mechanical linkages that separate the surgeon's hand from the tool, e.g., resector, at the distal (patient) end of the instrument by a long tube and a handle. The compression and elongation of materials used in instruments in this configuration prevent a linear relationship between the surgeon's hand movements and the tool's movements. This, in turn, causes positional uncertainty, disrupts the surgeon's tactile sense of how much pressure is being exerted by the tool, and impairs the surgeon's ability to perform dissections or delicate resections of tissue.

Another advantage of the invention is that the instruments are mounted directly on the surgeon's fingertips in a retractable or easily removable manner and a different instrument can be mounted on each of the surgeon's fingertips. Alternatively, when using the finger-sleeve and tool system, the surgeon can mount a single sleeve on a finger with a tool in a position that is most comfortable, e.g., either on the top, bottom, or side of his or her finger, and use only one sleeve. Typically a sleeve can be fitted onto the index finger or thumb of each, or either, hand. Either arrangement allows the surgeon to operate by hand within a cavity in the patient, and can avoid the need for repeated insertion and removal of the surgeon's hand through the wound.

Although avoiding repeated insertions of the surgeon's hand can reduce trauma to the patient and shorten the surgical procedure, modern hand entry ports prevent the escape of insufflation gas from the patient's cavity even after several insertions and removals of a hand. Further, the instruments' fingertip design, which allows the surgeon to bend all of the joints of his or her finger, provides the surgeon with maximum mobility.

In addition, the instruments can be designed so that the instrument can be retracted, either into the mount or instrument itself, or into the palm of the hand. This design prevents any accidental cutting or injuring of tissue when the surgeon uses his or her fingers to manipulate tissues, and when the surgeon inserts or removes his or her hand from a cavity within the patient.

In the detailed description which follows, a number of mechanisms for mounting surgical instruments on fingers and thumbs will be discussed.
Fig. 1 is an exploded schematic view of a fingertip-mounted minimally invasive surgical grasper embodying the present invention;
Fig. 2 is an isometric schematic view of the surgical grasper of Fig. 1 in the deployed position with the jaws in the open position.
Fig. 3 is a front schematic view of the surgical grasper of Fig. 2.
Fig. 4 is a plan schematic view of the surgical grasper of Fig. 2.
Figs. 5 is a cross-section side view of the surgical grasper of Fig. 2 sectioned along lines 5-5 in Fig. 4, and mounted on a fingertip.
Fig. 6 is a side schematic view of the surgical grasper of Fig. 2 in the deployed position with the jaws closed.
Fig. 7 is a side schematic view of the surgical grasper of Fig. 2 in the retracted position with the jaws closed.
Fig. 8 is an isometric schematic view of a fingertip-mounted minimally invasive suture needle holder embodying the present invention;
Fig. 9 is an isometric schematic view of a fingertip-mounted minimally invasive scissor embodying the present invention;
Fig. 10 is an isomeric schematic view of the fingertip-mounted minimally invasive scissor of Fig. 9 in a reverse angle view.
Fig. 11 is an isometric schematic view of an alternative harness for use in fingertip-mounted minimally invasive surgical instruments of the kind illustrated in Figures 1 to 10;
Figures 12 to 15 illustrate alternative means not in accordance with the invention, for mounting surgical instruments on a finger.
Figs. 12A and 12B are isometric schematic views of a flexible finger-sleeve mount used in conjunction with a miniature surgical grasper that is inserted into a pocket secured to the sleeve.
Figs. 13A and 13B are isometric schematic views of a closed-ended finger-sleeve mount used in conjunction with a surgical grasper, which includes a recess that is press fit onto a mounting rail projecting from the finger-sleeve.
Figs. 14A to 14C are isometric schematic views of fingertip-sleeves that are integrally connected to a miniature surgical grasper.
Figs. 15A to 15C are schematic diagrams of a one-piece wraparound finger-sleeve in which one end folds to form a pocket for accepting miniature surgical tools.
Figures 16 to 19 illustrate various wrap-around finger sleeves.
Figs. 16A and 16B are schematic diagrams of a top view (16A) and a side cross-sectional view (16B) of a dual-winged, wraparound finger-sleeve having a "butterfly" contour, and a centrally located miniature surgical tool pocket.
Figs. 17A and 17B are schematic diagrams of a wraparound, finger-sleeve having two wings that are connected by buttons or rivets, and includes a centrally located miniature surgical tool pocket.
Figs. 18A and 18B are schematic diagrams of a one-wing, wraparound finger-sleeve that is secured by adhesive, and includes a surgical tool pocket at the wide end.
Fig. 19 is a schematic diagram of a one-wing, wraparound finger-sleeve, that is secured to a finger by a mechanical interlocking of a tab and slots located on the wing.
Figures 20 to 31 illustrate a range of instruments that can be mounted on a finger and used in a surgical operation.
Figs. 20A and 20B are an isometric schematic view (20A) and a cross-sectional view (20B) of a fingertip-mounted minimally invasive surgical grasper, which can be used with a finger-sleeve.
Fig. 21 is a bipolar cord for providing electric current to miniature surgical instruments to provide an electrocautery feature.
Figs. 22A to 22D are a series of isometric (20A), end (20C), and cross-sectional (20B and 20D) views of a fingertip-mounted miniature surgical camera.
Figs. 23A to 23C are a series of an isometric view (23A), an exploded view (23B), and a partial cross-sectional view (23C), of a finger-tip mounted miniature surgical scissor.
Figs. 24A to 24C are a series of isometric schematic views of a fingertip-mounted electrocautery probe including a projecting mounting rail, that is used in conjunction with a finger-sleeve including a recess that accepts the projecting rail.
Figs. 25A and 25B are isometric schematic views of an electrocautery probe including a projecting mounting disk used in conjunction with a finger-sleeve including a circular recess that accepts the mounting disk. The finger-sleeve in Fig. 25B is shown in partial cross-section.
Figs. 26A to 26D are schematic illustrations of a thumb-mounted finger-sleeve including a projecting rivet or button, used in conjunction with a miniature surgical instrument that rotates from a deployed to a retracted position.
Figs. 27A to 27C are a series of isometric schematic views (27A and 27C), and a cross-sectional view (27B) of a thumb-mounted, miniature surgical scalpel.
Figs. 28A and 28B are a side schematic view (28A) and an isometric schematic view (28B) of a thumb-mounted, miniature surgical bipolar electrocautery grasper.
Figs. 29A to 29C are a series of schematic top (29A), side elevational (29B), and exploded (29C) views of a needle holder based on a cam design.
Fig. 30 is an isometric schematic view of a needle holder based on a toggle clamp design.
Figs. 31A and 31B are a pair of side views of a needle holder to be used in conjunction with a finger-sleeve mount, in open (31A) and closed (31B) positions.

The instruments described herein enable a surgeon to perform hand-assisted surgical procedures with his or her hand inside a patient's body cavity with all the benefits of minimally invasive surgery, but with the tactile feedback, control, and ease of manipulation of traditional, open surgery. In addition, the new instruments can be used in open surgery as well, since they can provide greater feedback and control than their traditional surgical instrument counterparts.

The instruments allow the surgeon to regain tactile feedback because, being mounted directly on the surgeon's fingertips, they require minimal actuation or linkage mechanisms. Further, by having the instruments mounted in such a way that the surgeon can manipulate them into a deployed or a retracted position with his or her thumb, the surgeon can easily use his or her fingertips to manipulate tissues. This ability to manipulate tissues without obstruction by the instruments is enhanced by the instruments' overall low profile design.

### Fingertip-Mounted Minimally Invasive Surgical Instruments

In general, any surgical tool or tip normally found on traditional surgical instruments or on laparoscopic or endoscopic instruments can be adapted for use in the present invention. For example, graspers, needle holders, clip appliers, dissectors, resectors, scalpels, scissors, and basket punches can be incorporated into the instruments of the invention. In addition, fittings for a variety of tubes or conduits, e.g., for irrigation and aspiration, and for electrical wiring, e.g., for monopolar or bipolar electrosurgical applications, can be added to the instruments of the invention.

The tools are secured to the surgeon's finger via a mount that is firmly attached to the finger or to a glove worn over the finger. The mount can take several different forms. For example, the mount can be a harness that is permanently connected to a particular tool, or the mount can be a separate generic mount, such as a finger-sleeve, that accepts multiple different tools.

Each harness-mounted instrument includes several main components: (1) a harness that is used to mount the instrument to the surgeon's fingertip; (2) a tool, such as a grasper or scalpel; and (3) a retracting/deploying mechanism attached to the tool that allows the tool to be manipulated into either a deployed position for surgery, or a retracted, locked position for fingertip manipulation of tissues. The retracting/deploying mechanism is attached to the harness, e.g., by an attachment mechanism, or is designed as a part of the harness. In certain embodiments, a portion of the tool may be an integral part of the retracting/deploying mechanism when the tool has two operating surfaces, e.g., as in a grasper or scissors.

Each of the tool and separate finger mount systems also include several main components: (1) a generic finger mount, such as a finger-sleeve, that includes a finger contacting portion and an attachment mechanism used to mount interchangeable tools to the surgeon's fingertip; and (2) a tool such as a grasper or scalpel. The tool can be attached to the mount by a connector in such a way that it can be easily removed. In certain embodiments, a portion of the tool may be inserted into a pocket on a finger-sleeve, for example, when the tool has two arms, e.g., as in a grasper. In other embodiments, the tool can include a clip that inserts into the finger-sleeve pocket, a recess that engages a protruding member on the mount, or a protruding member that engages a recess on the mount.

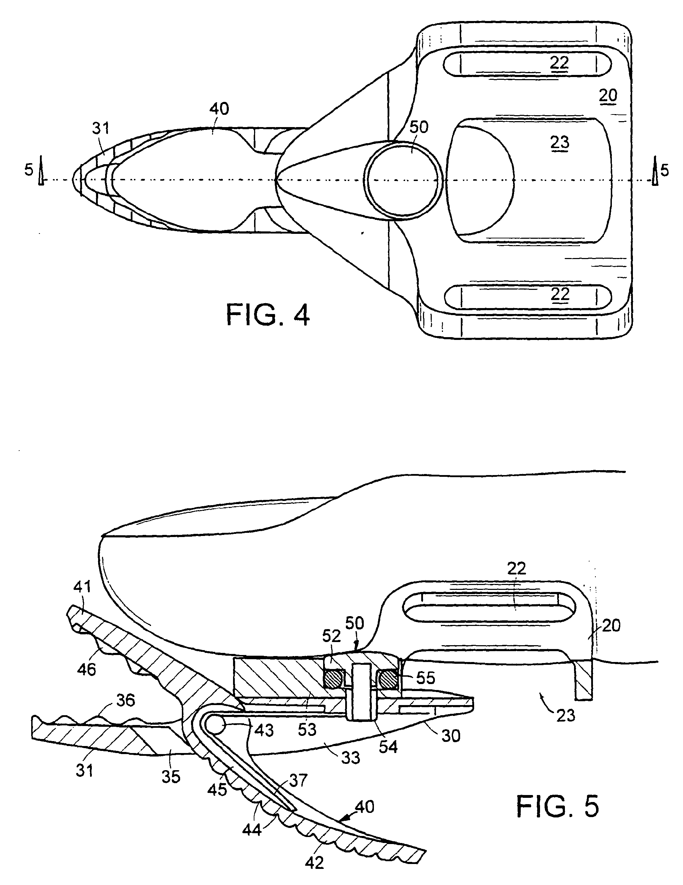

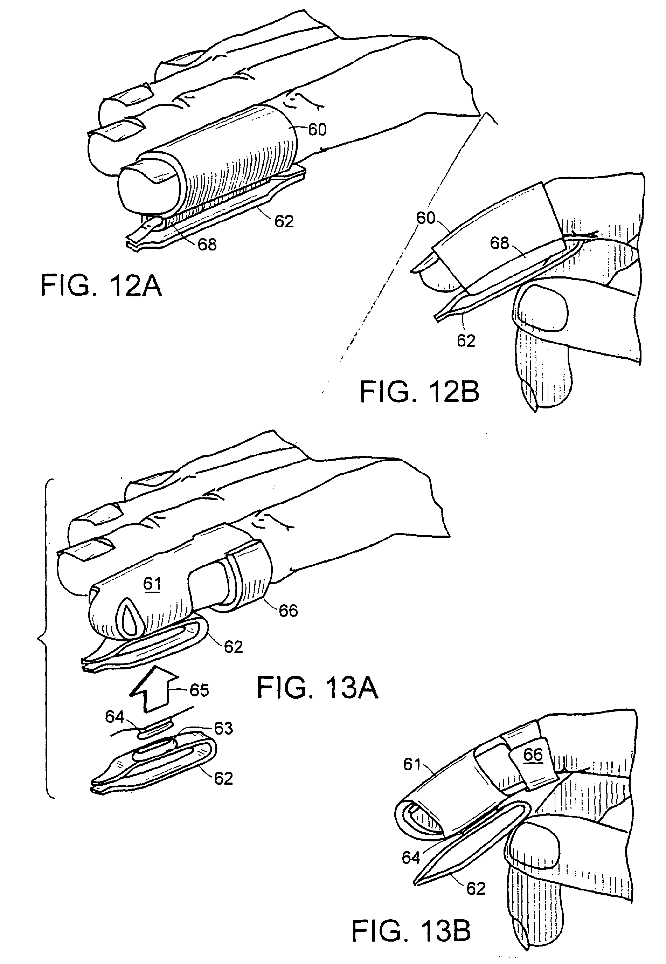

All of the instruments also share certain features. For example, they all have a low overall profile, especially in the closed, retracted position, and are designed to fit on the fingertip above the first joint (as shown in Fig. 5), or the second joint (as shown, e.g., in Fig. 12A). Further, in certain embodiments, e.g., where the mount is a harness, the retracting/deploying mechanism is preferably designed to rotate or swivel the tool from the deployed to the retracted position. This design allows for maximal extension of the tool beyond the harness in the deployed position while preserving the maximum mobility of the fingertip when the instrument is in the retracted position. However, the retracting/deploying mechanism can also be configured to slide the tool from the deployed to the retracted position in parallel to the harness. Further, the retracting/ deploying mechanism is designed to lock the tool into a safe, closed position when the instrument is retracted.

### Harness-Mounted Instruments

The concepts of one type of fingertip-mounted minimally invasive surgical instruments, the so-called "harness-mounted" instruments, will now be described in detail with respect to several specific instruments.

### Grasper

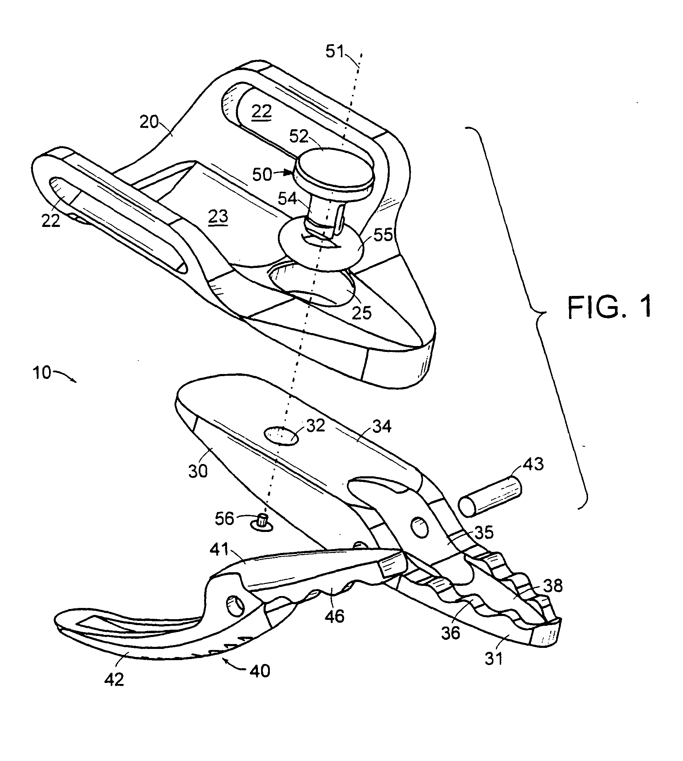

Fig. 1 shows an exploded view of one type of fingertip-mounted surgical instrument, a grasper 10, that can be used to manipulate or retract tissues, depending on the nature of the teeth in the jaws as described in further detail below. The grasper has three main parts, a harness 20; a retracting/deploying mechanism 30 integrally connected to a "stationary" jaw 31 (which is part of the grasper "tool" in this embodiment); and a moving component 40, which comprises a moving jaw 41 (which is another part of the tool) and an actuator 42. An attachment stud 50 (which is the attachment mechanism in this embodiment) connects harness 20 and retracting/deploying mechanism 30 so that they can rotate with respect to each other. The stationary jaw and retracting/deploying mechanism can be manufactured in one piece, or as two pieces that are rigidly connected. The same holds true for the moving jaw and actuator, which together form moving component 40.

The harness can be made from surgical grade stainless steels or, to decrease weight and manufacturing costs, the harness also can be made from a medical grade, rigid plastic material, e.g., ULTEM^{®} brand polyetherimides, or RADAL^{®} brand polyethersulphones. The harness is preferably a low cost, injection-molded component.

The materials used to manufacture the retracting/deploying mechanism 30, actuator 42, and stationary 31 and moving jaws 41 can vary depending on whether the instrument is best suited as a disposable or a reusable instrument. For reusable instruments, the jaws are made of robust, autoclavable materials such as surgical stainless steels or titanium. For disposable instruments, the jaws can be manufactured of cast alloys, metal injection-molded alloys, or medical grade plastics such as polyethersulphones and polyetherimides, optionally with metal inserts, e.g., for the gripping surfaces or for rigid internal ribs or struts.

Stud 50 is made of high strength surgical stainless steel, plastic, or a light-weight, insulating ceramic.

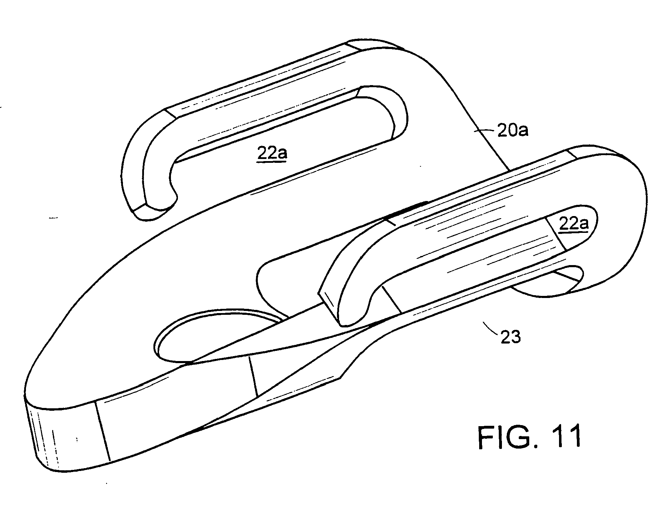

Harness 20 has a pair of cutouts 22 to allow a strap to be passed through the harness and secure the harness to a surgeon's fingertip. For example, the strap could be made of a hook and loop material, e.g., VELCRO^{®}, to loop around and wrap the finger to anchor the harness, and thus the entire instrument, on the fingertip. Fig. 11 shows an alternative harness design in which harness 20a includes cutouts 22a that are open at one end to simplify insertion or removal of a strap or webbing. In other respects this harness 20a is similar to harness 20 shown in Fig. 1. Both harnesses 20 and 20a further include cutout 23 so that as the strap is tightened, the surgeon can feel the strap on the lower portion of the finger to judge whether the harness is properly secured. Cutout 23 also provides easier access for the surgeon's thumb in rotating the retracting/deploying mechanism 30.

Stationary jaw 31 is so named because it does not move with respect to moving jaw 41, while moving jaw 41 moves with respect to jaw 31. In this embodiment, stationary jaw 31 is an integral part of the retracting/deploying mechanism 30. Thus, both parts move, because mechanism 30 is rotatably attached to harness 20 with attachment stud 50, as described in further detail below. Moving jaw 41 moves because it is rotatably attached to stationary jaw 31 with pivot pin 43.

Stud 50 passes through compression member 55 and a counterbored opening 26 in harness 20, and into hole 32 in retracting/deploying mechanism 30. Compression member 55 is seated on shoulder 53 (Fig. 5) at the bottom of opening 26. Member 55 can be a commercially available O-ring, e.g., made of an elastic material that is suitable for autoclaving (when used in a reusable device), such as polypropylene or silicon, or a commercially available spring, e.g., a helical stainless steel spring. Stud 50 includes a cap 52 and a hollow shaft 54. Shaft 54 can be split at the end opposite the cap as shown in Fig. 1, to allow the shaft to be radially expanded, after insertion into hole 32, to secure the end of shaft 54 in hole 32, e.g., with an insertable pin 56, and, preferably fixed with an adhesive or silver-solder. This arrangement secures retracting/deploying mechanism 30 to harness 20, while allowing rotation of one with respect to the other around central axis 51 of stud 50.

Cap 52 of stud 50 seats almost flush with harness 20 so that it does not protrude substantially above the inner wall of the harness, thereby avoiding any pressure on the surgeon's finger. However, since the inner wall of harness 20 is curved slightly to match the underside of the surgeon's finger, the slight protrusion of cap 52 provides the surgeon with tactile feedback as to whether the flat cap 52 of stud 50 is in its proper position as stud 50 pistons up and down when the tool is moved between the deployed and retracted positions.

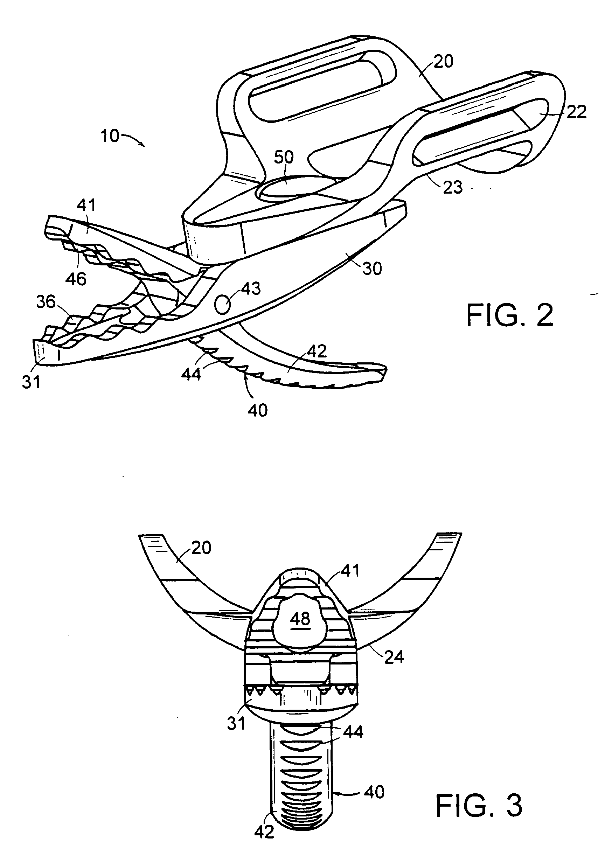

The outer surface of harness 20 is curved to form a convex mating surface 24 (as best seen in Fig. 3) that cooperates with a concave mating surface 34 on retracting/deploying mechanism 30. The radii of the curved mating surfaces, which are substantially the same for both surfaces, and the length of shaft 54 of stud 50 are adjusted to compress compression member 55 with cap 52 of stud 50 against shoulder 53 when surfaces 24 and 34 are aligned in parallel, e.g., as shown in Fig. 3, and to provide a maximal compression of ring or spring 55 when surfaces 24 and 34 are rotated out of alignment around axis 51.

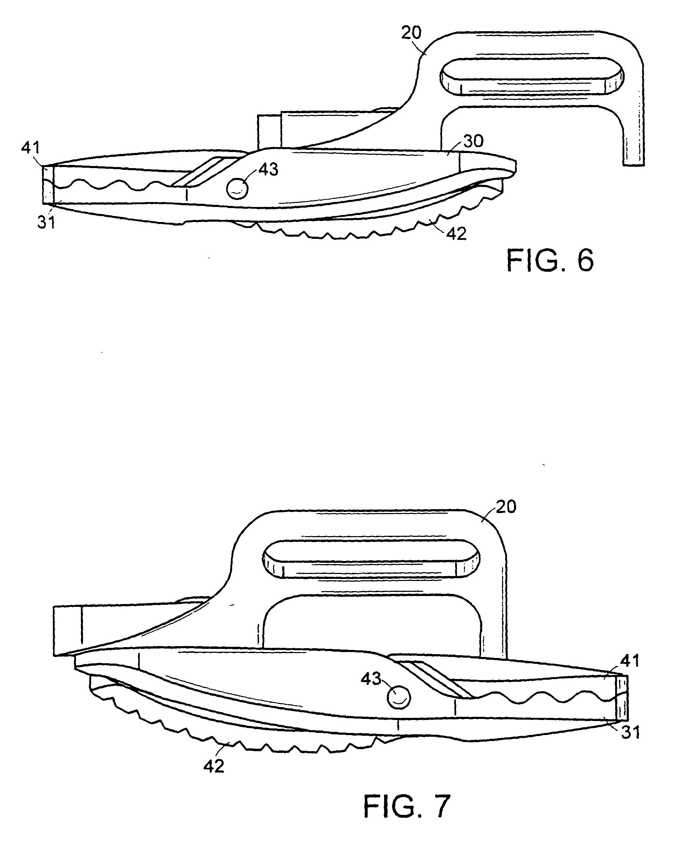

This arrangement allows the surgeon to use his or her thumb to rotate both stationary jaw 31 and moving jaw 41 of the grasper tool from a deployed position, as shown in Figs. 2 through 6, to a retracted position, as shown in Fig. 7. Compression member 55 provides a constant biasing force that keeps mating surfaces 24 and 34 passively locked in parallel alignment, either in the deployed or retracted positions, until the surgeon applies an actuating force greater than the constant biasing force with his or her thumb to rotate the grasper tool into the desired position.

The additional compression on member 55 serves as an over-center mechanism that biases retracting/deploying mechanism 30 into either one of the locked, deployed or locked, retracted positions.

As shown in Fig. 2, pivot pin 43 secures moving jaw 41 to stationary jaw 31. The pivot pin is made of a high strength steel and is either welded, soldered, press-fit, or orbitally riveted in place. The outer surface of actuator 42 of moving component 40 can include a textured or ribbed surface 44 to provide a better grip and thus more control for the surgeon. Furthermore, actuator 42 is shaped to have two curves that allow the surgeon to comfortably actuate the instrument from a variety of different angles, and to accommodate a variety of different hand sizes. The first curve runs along the longitudinal axis of actuator 42 as shown in, e.g., Fig. 5. The second curve is in the cross-section perpendicular to the longitudinal axis of the actuator, as best seen in Fig. 3.

Gripping surfaces 36 and 46, on stationary jaw 31 and moving jaw 41, respectively, include gripping teeth or gripping and cutting teeth. In the embodiment shown in the figures, the gripping surfaces are designed to manipulate within minimal injury those tissues and organs that are intended to be left in place, and thus include atraumatic, rolled teeth. However, when the gripping surfaces are designed to grip tissues tightly, without regard to injury, e.g., tissues that are to be resected, these teeth are designed as sharp points to provide greater bite or purchase into those tissues.

Gripping surfaces 36 and 46 are formed along the edges of their respective jaws, and include an empty space or fenestration 38 (Fig. 1) and 48 (Fig. 3) milled into the stationary and moving jaws, respectively. This configuration allows the gripping surfaces to exert a more positive grip on tissue. In a disposable device, the jaws can be made of a medical grade plastic, while the gripping surfaces can be made as metal inserts. Alternatively, metal inserts can be molded into the full length of a plastic jaw to provide strength and rigidity, or the entire jaw can be manufactured using metal-injection-molding (MIM) techniques.

As shown in Fig. 5, cavity 33 in retracting/deploying mechanism 30 allows actuator 42 of moving component 40 to be seated flush when the grasper tool is in the closed position as seen in Figs. 6 and 7. A large portion of the lower section of moving component 40 is actually housed within retracting/deploying mechanism 30 so that the instrument maintains an overall low profile, primarily so that when the instrument is in the retracted position (Fig. 7) it does not impede the surgeon's use of his or her fingertips.

Cavity 33 also provides a space for one end of a return spring 37 which continuously biases moving jaw 41 into an open position. Spring 37 can be a torsion spring, which can be wound around pivot pin 43 and can be made of, e.g. 0.0152 to 0.0381 cm (0.006 to 0.015 inch) diameter, high strength stainless steel wire. The spring can be made to have one, two, or three coils, or can be designed as a flat piece of spring steel with no coils. As shown in Fig. 5, the other end of the torsion spring can be seated lengthwise in milled cavity 45 within actuator 42 to provide the maximum return leverage. As an alternative, small magnets can be inserted into the jaws to use magnetic repulsion to keep jaws 31 and 41 biased in an open position.

Fig. 5 also shows a clearance cutout 35 in stationary jaw 31, which allows moving component 40 to be assembled into stationary jaw 31 by passing the moving component down, into, and through the cutout and into place, and then securing the two jaws with pivot pin 43.

The components of the surgical grasper are dimensioned to fit onto a surgeon's fingertip, as shown in Fig. 5. Thus, in a typical configuration, the harness is approximately 2.54 cm (one inch) or less in overall length so that it can fit on the first joint of the finger. The radius used on outer surface of the harness is about 1.016 cm (0.4 inch), and the overall height of the harness is about 0.762 cm (0.3 inch). The integral retracting/deploying mechanism 30 and stationary jaw 31 are approximately 2.921 cm (1.15 inches) long, have an overall height of approximately 0.469 cm (0.185 inches), and a width of approximately 0.660 cm (0.260 inches). The moving component 40, including actuator 42 and moving jaw 41, is approximately 2.514 or 2.540 cm (0.99) or (1.0 inch) in overall length, has an overall height of approximately 0.508 cm (0.2 inches) and a width comparable to that of the stationary jaw.

### Needle Holder

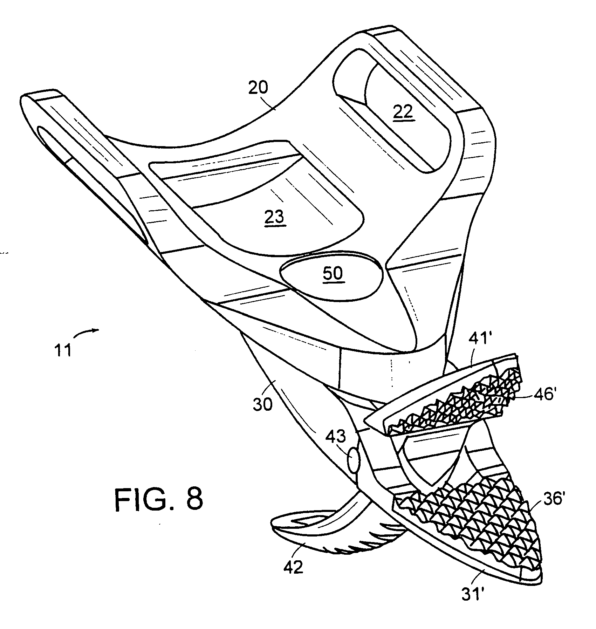

The suture needle holder is one of several instruments that are similar in general configuration to the grasper described above. As shown in Fig. 8, this instrument 11 includes harness 20, stationary jaw 31', moving jaw 41', and attachment stud 50. The harness and stud are the same as in the grasper described above, and can be manufactured in the same way as well. The overall dimensions are similar to those of the grasper. The retracting/deploying mechanism 30 can also be the same as in the grasper, but is integrally connected to a modified stationary jaw 31'. This jaw 31' can have a somewhat narrower design than jaw 31 in the grasper, but it also can be identical in size. The needle holder also has a spring (not shown) to bias the jaws in the open position, and the moving and stationary jaws are connected by pivot pin 43.

The main difference between the grasper and the needle holder is the nature of the gripping surfaces 36' and 46' of stationary jaw 31' and moving jaw 41', respectively, in the needle holder. These gripping surfaces, e.g., in the form of inserts, must be manufactured of a very hard material such as tungsten carbide steel, and preferably have a textured or knurled surface as shown in Fig. 8. These inserts can be of the same material and manufactured in the same way as the gripping surfaces of commercially available needle holders (e.g., those made by Aesculap A.G., Tuttlingen, Germany).

In another embodiment, the jaws of different instruments can be designed to be identical, and the gripping surfaces can be designed as interchangeable inserts. Thus, the jaws can be designed with fenestrations as in the grasper, and teeth inserts (e.g., as shown in Figs. 2 and 5) can be replaced with needle holder inserts (as shown in Fig. 8) which cover the fenestrations and provide a wide surface area to hold the needle securely.

As another feature, the needle holder can be manufactured to include a locking mechanism commonly referred to as a ratchet. Such a locking mechanism allows the surgeon to grasp a suture needle and feel the instrument clicking into a ratcheting, locked position that prevents the jaws of the instrument from opening until the surgeon applies pressure to undo the locking ratchet.

### Scissors

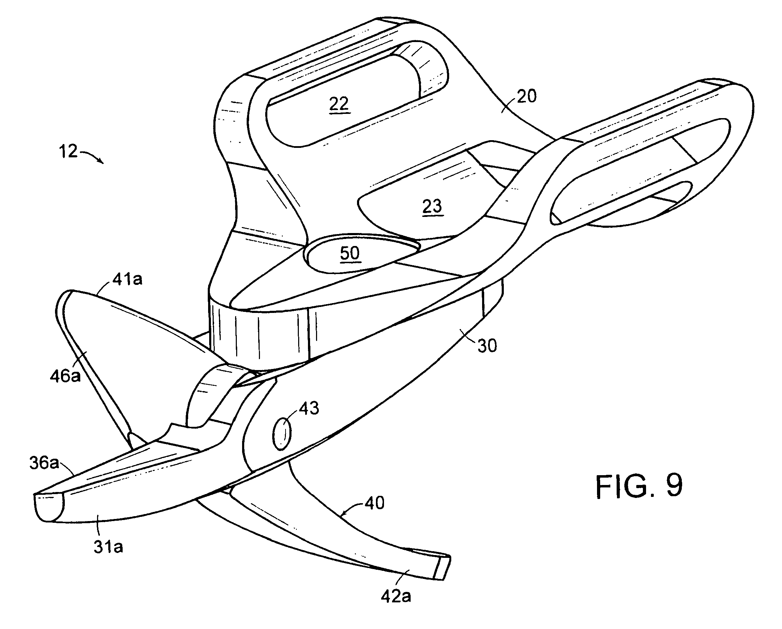

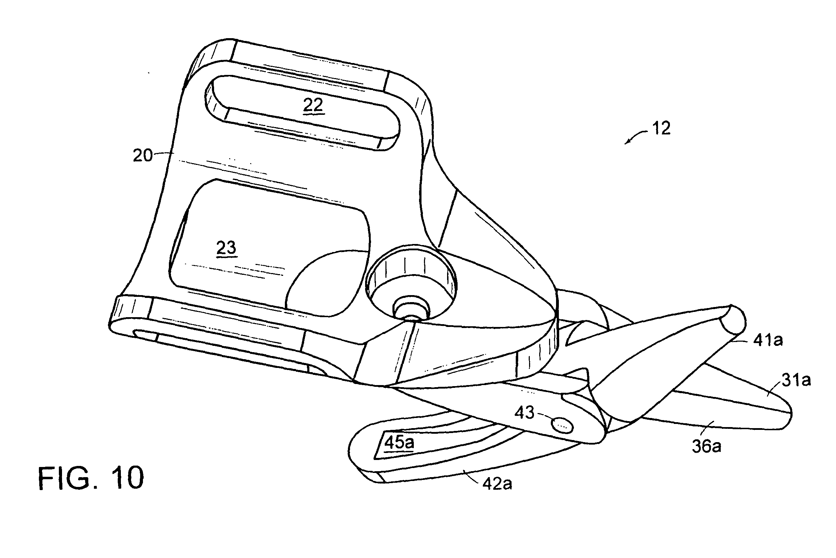

Scissors are another example of an instrument that is similar in general configuration to the grasper described above. As shown in Figs. 9 and 10, this instrument 12 includes a harness 20, stationary jaw 31a, moving jaw 41a, and attachment stud 50. The harness and stud are the same as in the grasper described above, and can be manufactured in the same way as well. The overall dimensions of the scissors are similar to those of the grasper. The retracting/deploying mechanism 30 is also the same as in the grasper, but is integrally connected to a modified stationary jaw 31a. This jaw 31a is modified to include a cutting edge 36a that cooperates in a shearing motion with a cutting edge 46a on moving jaw 41a. The scissors also have a spring (not shown) seated in cutout 45a (Fig. 10) in actuator 42a, to bias the jaws, and their respective blades, in the open position. Moving jaw 41a and stationary jaw 31a are connected by pivot pin 43.

The main difference between the grasper and the scissors is the cutting edges 36a and 46a of stationary jaw 31a and moving jaw 41a, respectively, in the scissors. The jaws are designed so that the cutting edges move past each other in a shearing motion, rather than butt against each other in a clamping motion as in the grasper. These cutting edges, e.g., in the form of inserts, for example if the jaws are made of plastic, must be manufactured of a very hard material such as surgical grade stainless steel or ceramic. These inserts can be of the same material and manufactured in the same way as the blades of commercially available laparoscopic surgical scissors (such as those made by U.S. Surgical Corp., CT).

### Finger Mounts and Instruments

The concept of another type of finger mounted minimally invasive surgical system, one including a separate tool and finger mount, such as a finger-sleeve, will now be described in detail with respect to several specific instruments and finger mounts.

In general, the finger mount/tool system is illustrated in Figs. 12A and 12B, which show a finger mount in the form of a flexible, cylindrical, finger-sleeve 60 attached to the index finger of a right hand. The sleeve can be made of any flexible and/or elastic material such as rubber (e.g., neoprene), LYCRA^{®}, or rubber-plastic combinations (thermoplastic elastomers) such as SANTOPRENE^{®} (Advanced Elastomer Systems, Inc., Akron, Ohio), or other flexible fabrics and plastics. In this figure, the tool is a miniature surgical grasper 62 that is mounted to the sleeve 60 by inserting one arm of the grasper through a pocket 68. The grasper 62 is opened and closed by pressure of the thumb. The finger-sleeve can be attached to any finger of the right or left hand, including the thumb, and in each case, is sized to fit a particular finger.

The finger mount can be easily and completely removed from the finger when it is secured by the elastic properties of the mount, such as a neoprene or LYCRA finger-sleeve, or it can be permanently attached to a surgeon's glove, e.g., by an adhesive layer on the inside of the mount. Typically, a permanent mount will be used on the surgeon's non-dominant hand, while an easily removed mount will be used on the dominant hand, so that this hand can be totally unencumbered when the mount is removed. Of course, very small and/or flexible finger mounts as described herein can be permanently attached to a glove and still keep the hand essentially unencumbered. Such mounts can be attached to the side or top of a glove finger to be as unobtrusive as possible.

Figs. 13A and 13B show an alternative version of the flexible finger-sleeve. In this case finger-sleeve 61 has a closed tip and includes a projecting mounting rail 64, preferably with rounded edges, that engages a recess or groove 63 in a tool, such as a surgical grasper 62. Both finger-sleeves 60 and 61 can be made of a flexible material, such as neoprene, LYCRA^{®}, or SANTOPRENE^{®}, and can be made of one continuous piece of material, or can be made with perforations or cutouts to allow ease of mobility for the fingertip. Of course, the protruding rail can be a part of the miniature tool, and the recess or groove can be the attachment mechanism on the finger mount.

If desired, the finger-sleeve can include a securing strap 66, as shown in Figs. 13A and 13B, e.g., secured by hook-and-loop material, other mechanical means, or adhesive, which helps ensure that the finger-sleeve does not inadvertently slip off the finger. However, a finger-sleeve, such as sleeve 60, made of an elastic material, does not normally require such an extra strap 66.

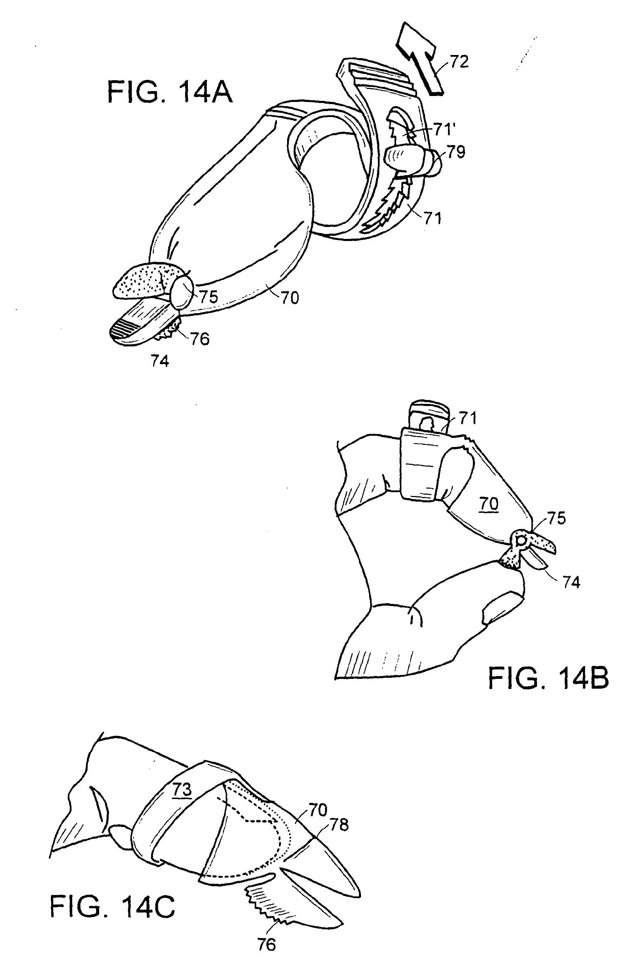

Another variation of the finger-sleeve is fingertip-sleeve 70, shown in Figs. 14A to 14C. Such a fingertip-sleeve can be worn on the dominant or non-dominant hand, and includes a tool, e.g., a miniature surgical grasper 74, integrally mounted on the tip of the fingertip-sleeve. As shown in Figs. 14A and 14B, the grasper can include a pivot pin 75 or can be manufactured of a plastic material having a living hinge 78 that connects the upper and lower jaws. In either embodiment, the lower grasper jaw is manipulated by an actuator 76, which may be formed with a ridged or otherwise textured surface. In this embodiment, the finger-sleeve can be made of a rigid plastic, since it covers only the fingertip above the first joint.

As best shown in Fig. 14A, the fingertip-sleeve 70 is secured to a finger with a strap 71 having an opening 71' that cooperates with a wedge 79 with tabs to secure and tighten the strap. In this particular embodiment, the opening 71' has ridges that engage wedge 79 to enable strap 71 to be tightened in the direction of arrow 72, but do not allow the strap to move in a loosening direction unless the tabs of wedge 79 are compressed.

In an alternative embodiment shown in Fig. 14C, flexible straps 73 can be manufactured of a malleable metal or wire, or a plastic or fabric in which wires are embedded (or laminated), such that they can be easily wrapped around a finger to secure the fingertip-sleeve.

### Finger-Sleeve Variations

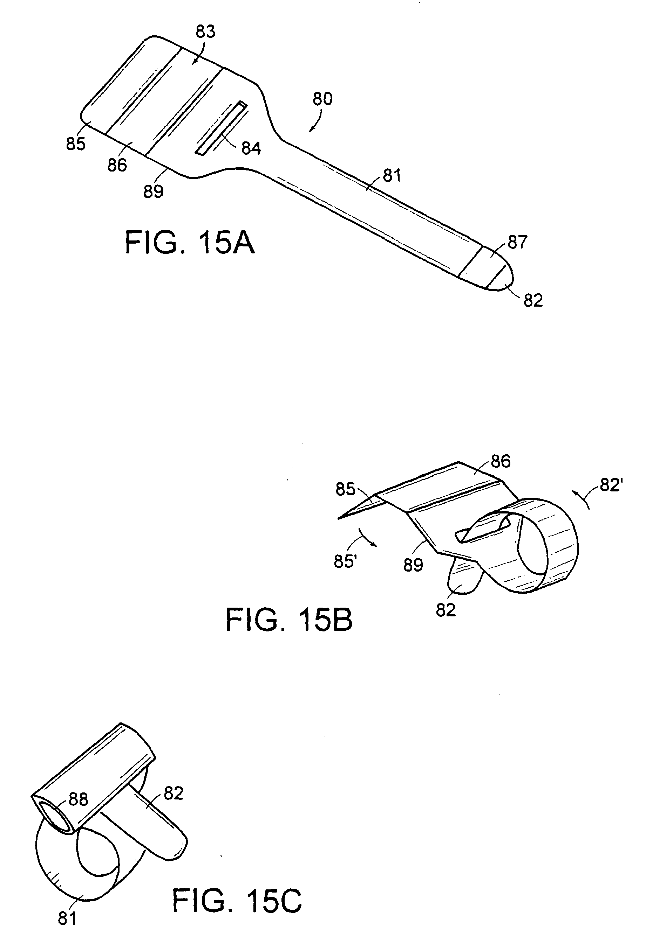

The finger-sleeve embodiments described above can be manufactured in a variety of configurations. For example, in Figs. 15A to 15C, show a one-piece, wraparound, finger-sleeve 80. Sleeve 80 can be stamped or cut out of a single piece of fabric, plastic, neoprene rubber, or SANTOPRENE^{®}, and then assembled, e.g., with adhesives, to form the completed sleeve. In use, portions 85 and 86 of proximal end 83 are folded in the direction of arrow 85' as shown in Fig. 15B, and secured to the back of portion 89 with adhesive, to form an internal pocket 88 as shown in Fig. 15C. To secure sleeve 80 to a glove finger, elongated portion 81 is wrapped around the finger, and distal end 82 is inserted through opening 84 and pulled tight around the finger in the direction of arrow 82' (Fig. 15B). An adhesive patch 87 is then used to secure distal end 82 to the appropriate location along elongated section 81 to make the sleeve fit snugly on the surgeon's finger. To permanently secure the mount to a glove finger, the underside (side facing the finger) of the elongated portion 81 can be glued directly to the glove finger.

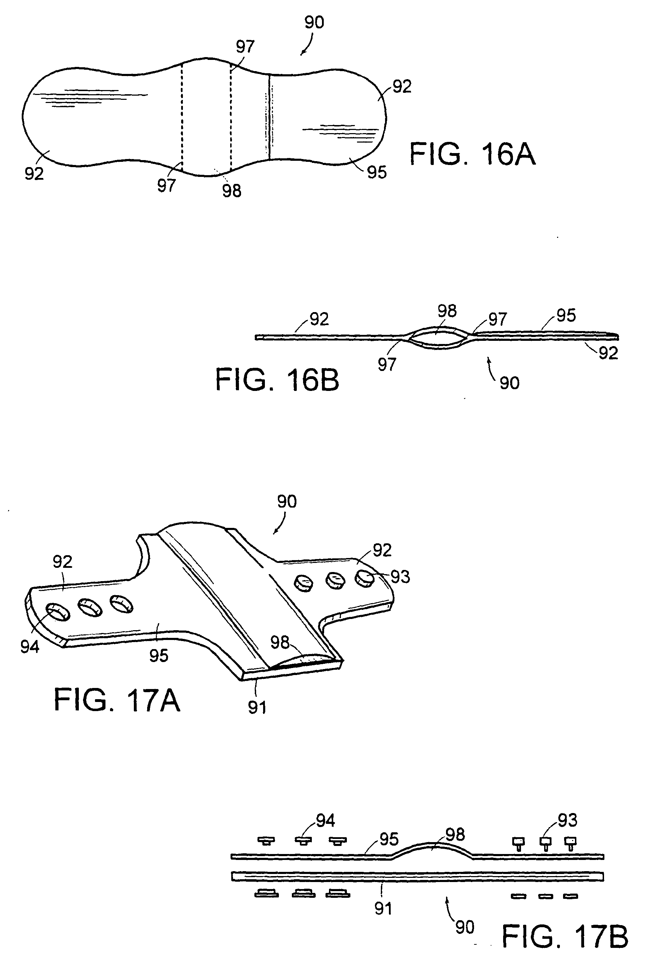

Fig. 16A shows a top view of a general, dual-wing, wraparound finger-sleeve 90. The outer contour has an approximate "butterfly" shape, which allows for greater freedom of motion of the finger once inserted into the sleeve. Sleeve 90 has two wings 92 and a centrally located pocket 98. This sleeve can be manufactured of plastic, fabric (LYCRA^{®}) or a flexible rubber, such as neoprene or SANTOPRENE^{®}. The pocket 98 can be formed by stitch lines 97, and the sleeve can be temporarily secured to a finger by wrapping one wing 92 over the other and securing with an adhesive patch 95. The adhesive can be, for example HIGH TACK 950^{®} (3M, Minnesota) or high performance adhesive transfer tape (3M, Part No. F9755PC). In addition, adhesive can be used to permanently secure the sleeve to a glove finger.

The finger-sleeve can be manufactured by extruding rubber or thermoplastic elastomers such as SANTOPRENE^{®} through an extrusion die having a shape similar to the cross-sectional view of Fig. 16B to form long sheets having a hollow "pocket" 98 in the center. The individual finger-sleeves are formed by stamping or cutting them out of the long sheets using a cutter having a contour in the shape of the top view shown in Fig. 16A. The finger-sleeves can also be manufactured by laminating sheets of different materials, and heat sealing the edges of the pocket.

The dimensions of such a dual-wing sleeve 90 can be, for example, approximately three inches in overall length (in an unrolled state), approximately one inch in the broadest width (e.g., in the area of pocket 98), and the pocket can be approximately 0.4 inches by 0.04 inches.

Figs. 17A and 17B show an alternative embodiment, in which a dual-wing, wraparound finger-sleeve is secured by mechanical means, for example, projecting snaps 93 that engage hollow openings 94 to secure the sleeve 90 to a finger. Again, a centrally located pocket 98 is used to secure a miniature surgical tool to the finger-sleeve. As shown in Fig. 17B, a top surface 95 of the finger-sleeve 90 can be made of a LYCRA^{®} and/or SPANDEX^{®} material, with an underlying support 91 made of neoprene. The LYCRA^{™} and neoprene materials can be laminated, and the snaps or projections 93 are riveted through the laminated material. These snaps can be made of a plastic such as polypropylene.

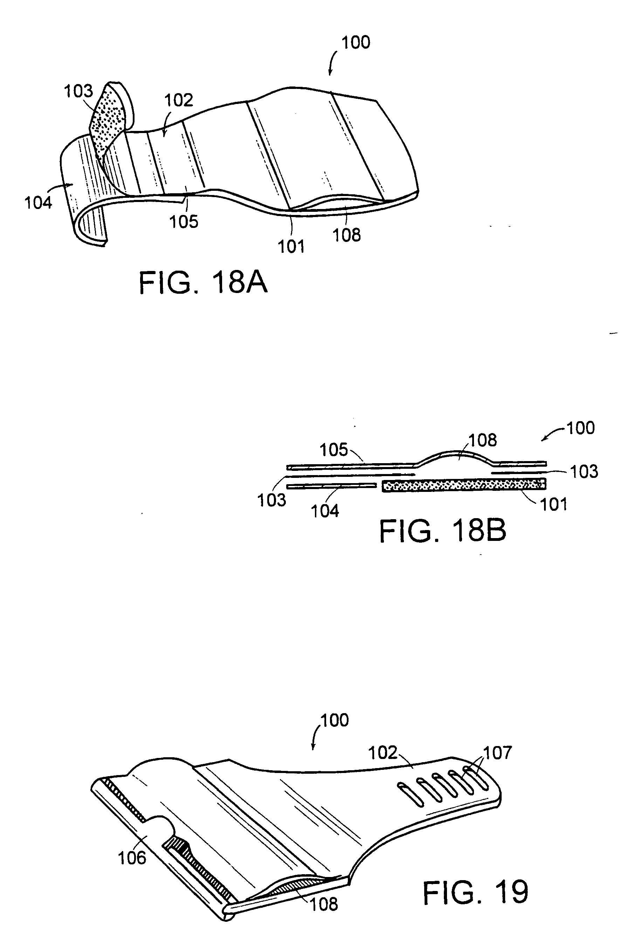

Figs. 18A and 18B show a one-wing, wraparound finger-sleeve 100 with one wing 102 and a pocket 108 located off-center, in a wide region of the sleeve. As best shown in Fig. 18B, the top surface 105 of sleeve 100 can be manufactured of a SPANDEX^{®} or LYCRA^{®} material laminated to a support 101 made of neoprene by an adhesive layer 103. The adhesive layer 103 secured to wing 102 has a non-stick backing 104 to protect it before use, which is removed just prior to securing the sleeve to a finger.

Fig. 19 shows an alternative embodiment of a one-wing, wraparound finger-sleeve 100. In this embodiment, wing 102 is wrapped around the user's finger and secured mechanically, by inserting tab 106 through the appropriate opening 107 to produce a secure fit.

Other finger-sleeve mounts can be designed based on the general principles described herein. For example, the finger-sleeve can be of the wraparound design, but can be secured to the finger not with adhesive or buttons, but with malleable wire built into, or secured to the surface of, the wings, so that the wings can be secured merely by wrapping the wings around the finger. In addition, a solid, yet flexible and resilient, plastic cylinder can be used to make a finger-sleeve that includes a slit along its entire length to enable it to be slipped over a finger, and then be secured by the spring action of the plastic cylinder walls. Other embodiments include "sleeves" made of two separate rings that slip over a finger and are connected by a flexible plastic hollow rod or tube into which a part of the tool (or a clip) is inserted during use.

### Finger-Sleeve Mounted Instruments

The finger-sleeve mounted instruments all have several features in common. The tools share a low overall profile in their design to fit on the fingertip above the first two joints, or on the thumb. In addition, each of the tools includes a component that can be inserted into a pocket on a finger-sleeve, or a connector that engages an attachment mechanism on the finger-sleeve. This component can be a part of the tool, e.g., one arm of a grasper, or a separate clip attached to the tool. Alternatively, the tool can include a projecting pin or rail, or a recess or groove, that corresponds to a recess or projecting pin or rail located on the finger-sleeve mount.

### Grasper

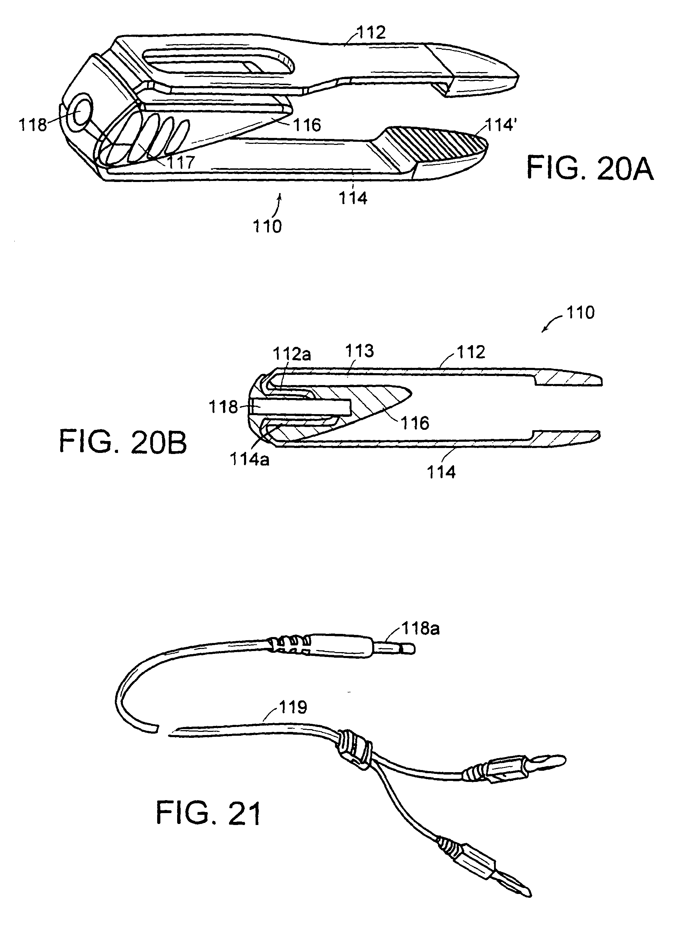

Figs. at 20A and 20B show a miniature surgical grasper for use with a finger-sleeve including a pocket. In this embodiment, grasper 110 includes two arms 112 and 114 having grasping tips at their distal ends, which include a textured surface, e.g., 114'. As best shown in Fig. 20B, arm 112 includes a proximal tab 112a which is inserted into a support 116, e.g., made of surgical grade plastic, e.g., by injection molding. Similarly, arm 114 includes a tab 114a, which is inserted into a slot in support 116. The support 116 can be provided with ridges 117 to allow the surgeon to easily manipulate the grasper 110. Tab 112a and support 116 are designed such that space 113 is created between support 116 and arm 112. This space has a dimension that allows arm 112 to be inserted easily and securely into the pocket of a finger-sleeve.

Grasper 110 is provided with a socket 118 for an electrical plug, to allow grasper 110 to be used as a bipolar electrocautery instrument. As can be seen in Fig. 20B, both of tabs 112a and 114a extend into socket 118, so that they contact two different portions of a plug 118a.

Fig. 21 shows a bipolar cord 119 with plug 118a that fits into socket 118. The cord can be coaxial. The plug has two segments separated by insulation, one segment to contact tab 112a and the other to contact tab 114a, and can be manufactured using standard techniques. A small diameter plug, e.g., on the order of 0.78 to 0.093 or even 0.125 inches, is required. The plug is made of metal or other conducting material, whereas the plastic around the plug (the strain relief) is preferably made of a flexible material to allow the surgeon ultimate mobility of his or her fingers.

The materials used to manufacture the grasper and all of its parts can vary depending on whether the instrument is best suited as a disposable or a reusable instrument. For reusable instruments, the jaws and arms are made of robust, autoclavable materials such as surgical stainless steels or titanium. For disposable instruments, the arms can be manufactured of cast alloys, metal injection-molded alloys, or medical grade plastics such as polyethersulphones, polypropylenes, and polyetherimides, optionally with metal inserts, e.g., for the gripping surfaces. The arms are preferably stamped or die-cut of a stainless steel sheet and then bent into the proper configuration. The tabs of the two arms are then inserted into a mold, and support 116 is injection molded around the metal tabs. Alternatively, the support can be manufactured separately, and the tabs inserted using a press-fit and/or adhesive.

The surgical grasper is dimensioned to fit onto a surgeon's fingertip, as shown in Fig. 12A. Thus, in a typical configuration, the grasper is approximately one and one half inches or less in overall length so that it can fit within the first two joints of the finger.

### Camera

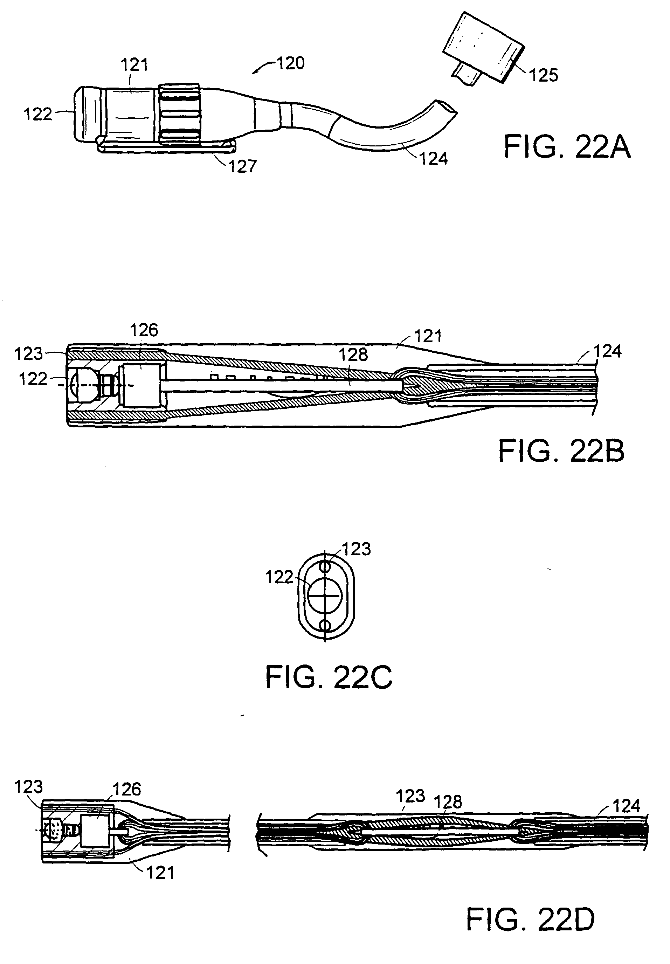

Another miniature surgical instrument that can be mounted on a finger-sleeve is a camera. Various camera embodiments are illustrated in Figs. 22A to 22D. Camera 120 is provided with a housing 121, an objective lens 122, and a clip 127 that fits into a pocket on a finger-sleeve. The camera projects light into the body cavity via optic fibers 123 and sends a video signal to the operator through cable 124.

Printed circuit board 128 converts analog signals to a digital video image. The camera can be a charge-couple device (CCD) including a CCD camera chip 126, e.g., a Panasonic CCD Chip (e.g., No. GP-KS462). The CCD camera is connected to a cable 124 that extends from the camera chip 126 and runs along the surgeon's hand and arm, e.g., to a camera control unit 125, e.g., including a light source, and monitor, outside the patient. The printed circuit board 128 can be either within cable 124, as shown in Fig. 22D, or within the housing 121, as shown in 22B. Fig. 22C shows an end view of camera 120, having an oblong configuration. In addition, circular or other cross-sections can be used.

The camera and light source can be supplied with electricity through cable 124 from a power source located outside the patient (see, e.g., Oz, U.S. Patent No. 5,079,629). Alternatively, light can be transmitted through optic fibers 123 located within cable 124 from a light source outside the patient. Signals from the CCD camera can be transmitted to a monitor through cable 124 or by an antenna (not shown), e.g., as described in Oz, U.S. Patent No. 5,079,629.

The fingertip mounted camera is typically assembled in a tubular housing and then sealed with an elastomeric or silastic material. The techniques to make such miniature cameras is known from the field of endoscopy.

### Scissors

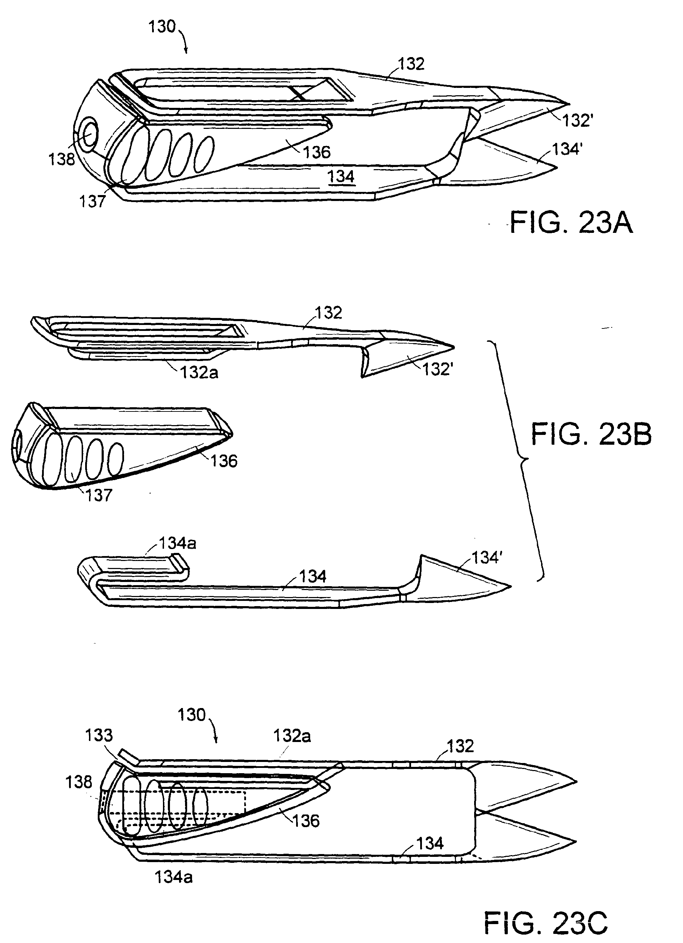

Scissors are similar in general configuration to the grasper described above with respect to Figs. 20A and 20B. Figs. 23A to 23C show miniature surgical scissors 130. Scissors 130 include two arms 132 and 134, with corresponding cutting blades 132' and 134'. As with the grasper described above, the two arms of the scissors are connected by a supporting structure 136 that can include ridges 137, or other textured surface to allow easy manipulation. Arm 134 includes a tab 134a which is inserted into support 136. Arm 132 includes a tab 132a, which is formed as a cut-out from the remainder of arm 132 as best shown in Fig. 23b. Tab 132a is inserted into support 136 in a manner to provide a space 133 between support 136 and arm 132 (Fig. 23C). Also as in the grasper, the arms can be molded directly into the support 136, e.g., by injection molding. Blades 132' and 134' are biased against each other to ensure a shearing motion, and to provide a natural spring effect biasing the blades into an open position.

Space 133 is open at the proximal end of the scissors 130, and arm 132 is thereby free to be inserted into a pocket on a finger-sleeve. Scissors are therefore inserted into a pocket from the distal end of the finger-sleeve, as opposed to the grasper described above, which is inserted from the proximal end of a finger-sleeve.

The cutting edges must be manufactured of a very hard material such as surgical grade stainless steel or ceramic. These blades can be of the same material and manufactured in the same way as the blades of commercially available laparoscopic surgical scissors (such as those made by U.S. Surgical Corp., CT). As in the grasper, support 136 is provided with a socket 138 that enables the scissors to be used as an electrocautery instrument. However, as can be seen in Fig. 23C, only tab 134a extends into socket 138, so the scissors provide monopolar electrocautery.

### Needle Holder

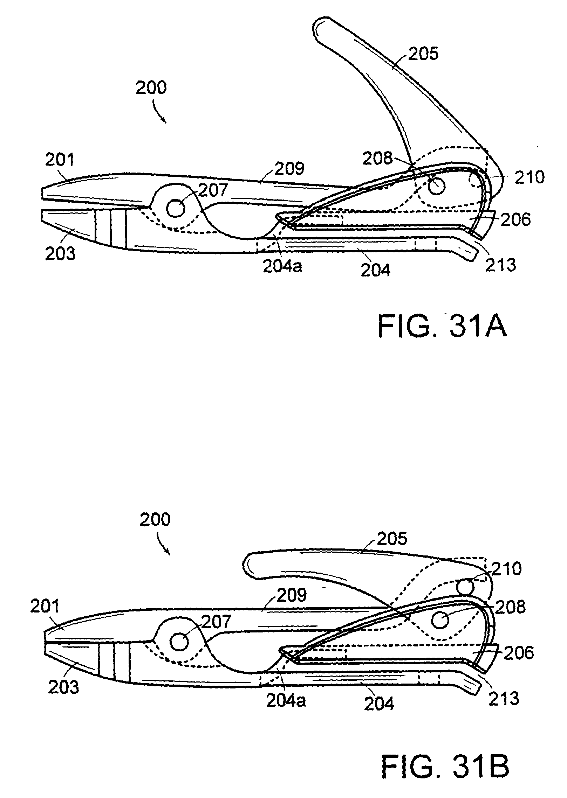

Figs. 31A and 31B show a needle holder designed for use with a finger-sleeve mount that includes a pocket to receive an arm of a tool or clip attached to a tool. In this embodiment, the needle holder 200 includes upper jaw 201, which is an extension of upper arm 209, and lower jaw 203, which an extension of lower arm 204, that are held together by pin 207. Upper jaw 201 is actuated by lever 205 through a number of cam surfaces and pins. In particular, lever 205 is connected to support 206 by pivot pin 208, and moves upper arm 209 by driving pin 210. Once lever 205 is in the closed position (Fig. 31B), the upper 201 and lower 203 jaws are also in a closed position, but the lever and two fulcrum system provides a significant force compounding effect to achieve a mechanical advantage.

The mechanical advantage arises from (1) the ratio of the length of jaw 201 beyond the pin 207 compared to the distance between pin 207 and driving pin 210, and then (2) the ratio of the length of lever 205 (the distance from its tip to pivot pin 208) compared to the distance between driving pin 210 and pivot pin 208. Great pressure is required to hold a needle in a needle holder, and the system shown in Figs. 31A and 31B amplifies the force that can be applied with a thumb, and thus provides the required force. If drive pin 210 is moved within lever 205 so that it is centered over pivot pin 208 in the closed position, or is just past center, then the needle holder will provide a closed and "locked" position, while, as illustrated in Figs. 31A and 31B, the surgeon must maintain pressure on lever 205 to maintain the needle holder 200 in the closed position.

The lower arm 204 is connected to support 206 by tab 204a, but as in the scissors (Fig. 23C), is separated from support 206 by space 213, which enables lower arm 204 to be inserted into a pocket of a finger-sleeve.

### Alternative Finger Mounts

In addition to the finger-sleeve mounts described above, other finger mounts are included in the present invention. Each of these mounts are easily secured to a finger to fasten a miniature surgical tool to the fingertip for use, and are easily removed when the tool is no longer required. These mounts are secured to the fingertips by mechanical means or adhesive, and can form complete circles around the finger, like a ring or sleeve, or form semicircles, that are secured by means of straps, webbing, adhesive, or adhesive tape, e.g., surgical tape.

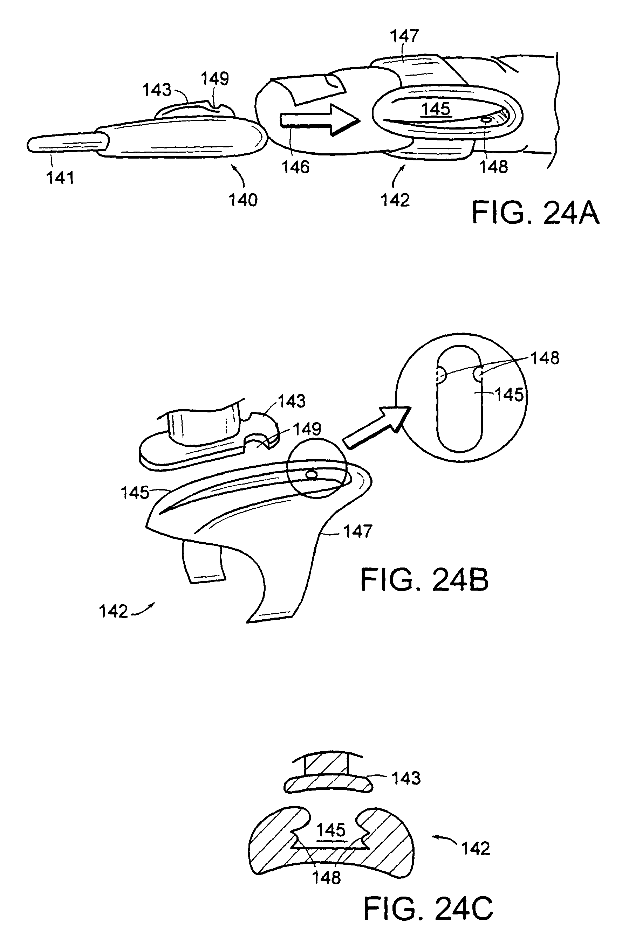

One example of an alternative finger mount is illustrated in Figs. 24A to 24C. In this embodiment, finger mount 142 is similar to harness 20 in Fig. 1, but includes a recess 145 designed to receive a projecting rail 143 that extends from a miniature surgical tool, such as an electrocautery probe 140 having a cauterizing tip 141, shown in Figure 24A. Tool 140 slides in the direction of arrow 146 to secure rail 143 into recess 145 by means of detents 148 that engage notches 149 in rail 143. An enlarged view of the recess and detents 148 is shown in Fig. 24B. Fig. 24C shows a cross-sectional view of recess 145 in finger mount 142, as well as rail 143 which projects from a tool.

Mount 142 is secured to a finger by means of wings 147, which can be made of a solid plastic that is dimensioned to securely fit onto a particular finger. Wings 147 can be made resilient, to provide a secure, press-fit, or can be made of a fabric or other material that can be secured by, e.g., surgical tape, or mechanical means such as snaps or buttons. Further, wings 147 can be glued directly to a finger of a glove on a surgeon's hand.

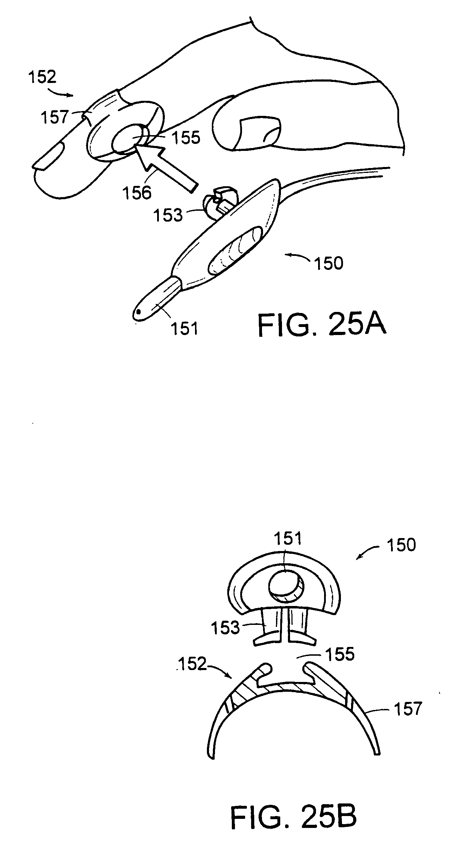

Figs. 25A and 25B show an alternative embodiment, in which a tool 150, e.g., an electrocautery probe having a cauterizing tip 151, is provided with a projecting disk 153 that is inserted into holder 152 in the direction of arrow 156 (Fig. 25A). As shown in Fig. 25B, holder 152 has two wings 157 which form a semicircle corresponding to the contour of the finger. This mount 152 can be secured by, e.g., an adhesive or an adhesive tape. As shown in Fig. 25B, projecting disk 153 is formed of two separated halves, that can be pressed together to allow the disk to be inserted into recess 155 in mount 152 to form a press-fit engagement. Other mechanisms to attach the tool to the mount can be used and easily designed.

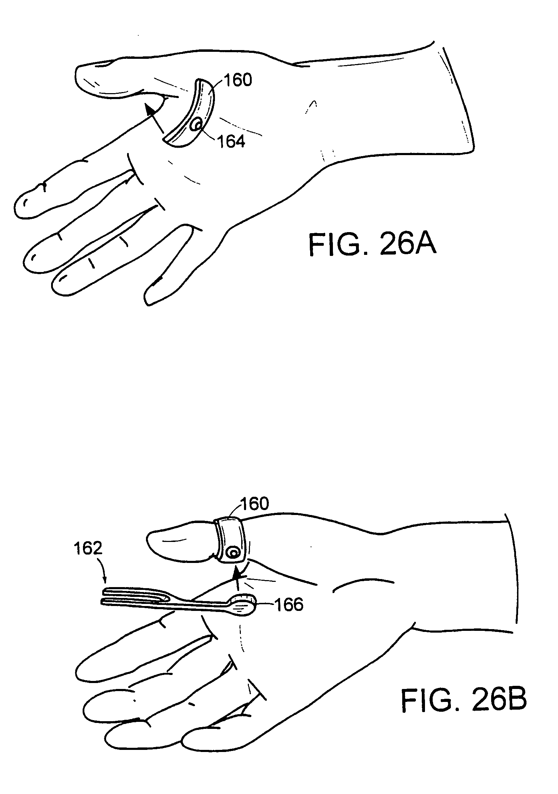

Another alternative embodiment of the finger mounts is shown in Figs 26A to 26D, which illustrate a thumb ring finger mount 160, that is secured to the thumb by means of surgical tape, adhesive, or other mechanisms described herein. Mount 160 is provided with a rivet or projecting pin 164 that cooperates with a snap connector at the proximal end 166 of a miniature surgical tool 162 (as an example, a grasper is shown in Figs. 26B to D). This pin 164 can be provided with an electrical cable to provide any instrument attached to the pin with an electrocautery capability.

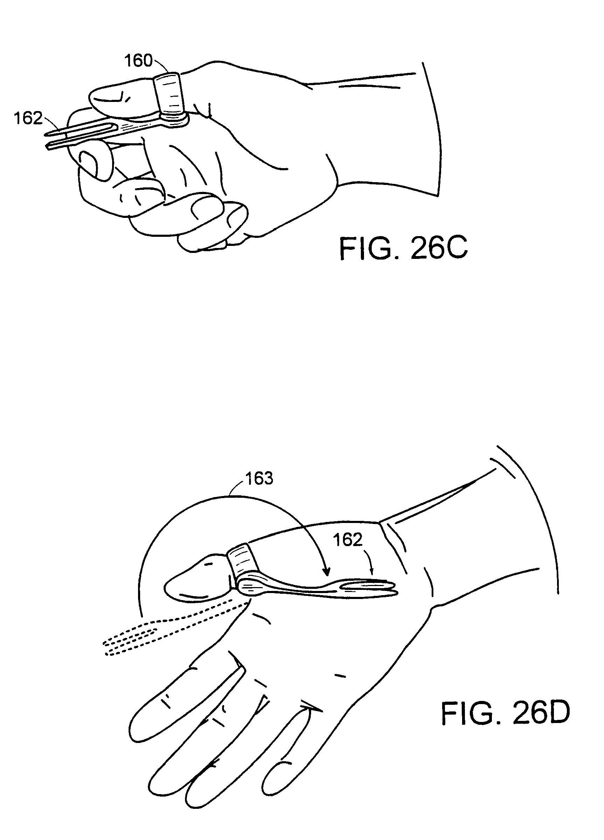

Fig. 26C shows grasper 162 in the deployed position, whereas Fig. 26D shows the motion of grasper 162 along arrow 163 into a retracted position, in effect, hidden within the surgeon's palm.

### Thumb-Mounted Grasper

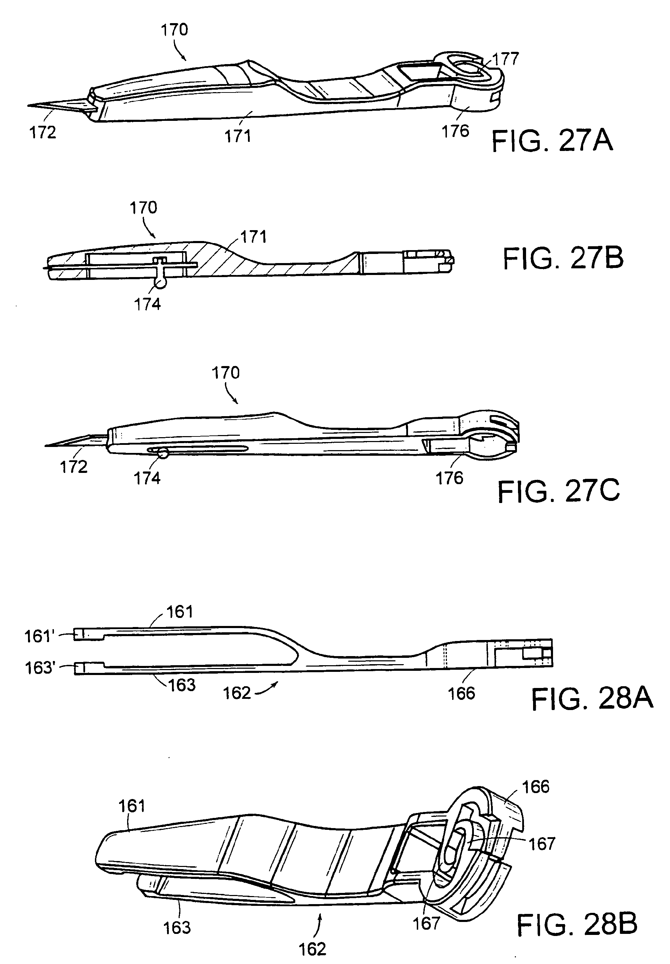

Figs. 28A and 28B show grasper 162 in closer detail. Although this grasper is designed for use with a thumb mount, it can also be used with a finger mount having a projecting pin or rivet. Grasper 162 includes grasper arms 161 and 163 at the distal end, as well as electrocautery tips 161' and 163'. Electricity for electrocautery can be supplied by an electric cord (not shown). The distal end 166 of grasper 162 is provided with a connector snap mechanism that allows the grasper to be securely fastened to the projecting rivet or pin 164 located on finger mount 160. When pressure is applied to both sides of distal end 166, two curved arms 167 are brought further apart to allow the pin to be inserted between them and secured once pressure is release allowing the two arms 167 to snap together around the pin 164. This grasper can be made of a shape-memory alloy metal, or a rigid plastic material using standard techniques. Useful dimensions for this and other thumb-mounted tools are about 7.62 cm (3 inches) in length, and an overall width of from about 0.635 to 0.762 cm (0.25 to 0.3 inch)

### Thumb-Mounted Scalpel

An alternative miniature surgical instrument for use with the thumb mount 160 is a scalpel 170 shown in Figs. 27A to 27C. Again, a regular finger mount can be used. Scalpel 170 includes a scalpel blade 172 that can be retracted into the housing 171 of scalpel 170 by means of an actuator 174. The distal end 176 of scalpel 170 is secured to the projecting pin 164 of mount 160 with curved arms 177, in the same manner as described for grasper 162. Fig. 27B shows a cross-sectional view of a simple deploying and retracting mechanism within scalpel 170, whereas Fig. 27C shows a bottom isometric view of scalpel 170 in the deployed position. The deploying and retracting mechanism is designed to lock the scalpel into either the deployed or retracted positions, and can be manufactured using standard techniques.

### Thumb-Mounted Needle Holder

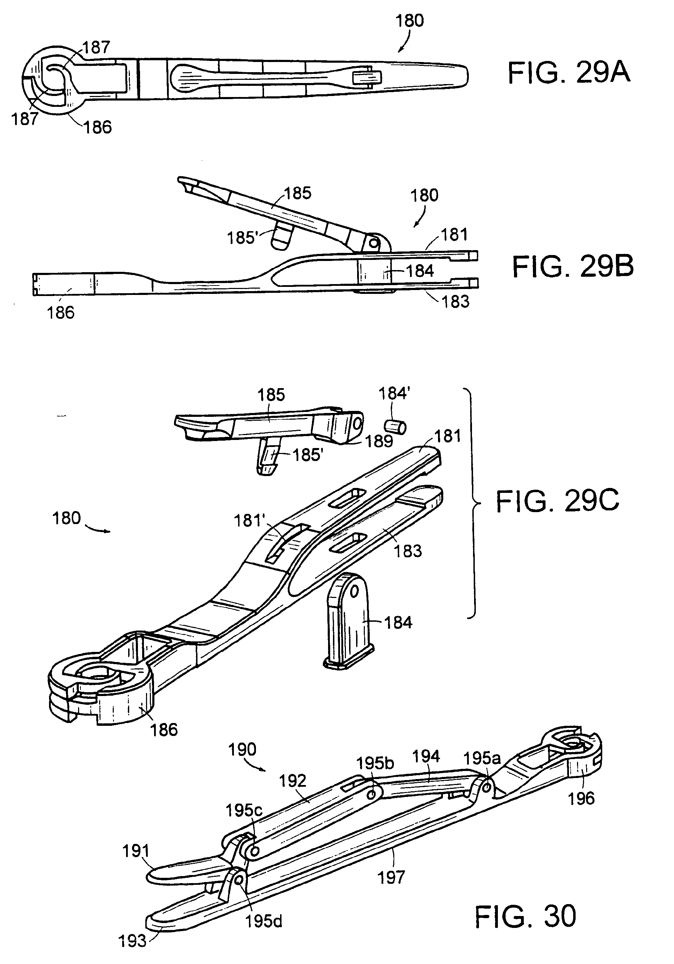

Figs. 29A to 29C show a needle holder designed for use with a thumb mount 160 as shown in Figs. 26A to 26D. The proximal end 186 includes curved arms 187 that snap around pin 164 of mount 160. The distal end of the needle holder 180 is very similar to the grasper 162 shown in Figs. 28A and 28B, but includes certain additional components that allow the arms 181 and 183 to be closed in a locked position. These components include a fulcrum pin 184, a lever 185 connected to fulcrum pin 184 with an assembly pin 184', and a locking hook 185' that engages a notch 181' when arms 181 and 183 are in a closed position.

Lever 185 includes a cam surface 189 that causes arms 181 and 183 to be pushed together as lever arm 185 is pressed downwardly. Once the arms are closed, e.g., on a needle, further pressure on lever 185 causes hook 185' to engage notch 181' and the surgeon can let go of the lever and be confident that the needle is held firmly in place. Additional side pressure on lever 185 causes hook 185' to disengage, allowing the arms 181 and 183 to open, releasing the needle.

All components of the needle holder are preferably made of surgical grade stainless steel. The distal ends of arms 181 and 183 are preferably provided with gripping surfaces, e.g., in the form of inserts, that are manufactured of a very hard material such as tungsten carbide steel. These inserts can be of the same material and manufactured in the same way as the gripping surfaces of commercially available needle holders (e.g., those made by Aesculap A.G., Tuttlingen, Germany).

Fig. 30 shows an alternative embodiment of a needle-holder 190 that can be used with a thumb mount 160. The proximal end 196 of the needle-holder 190 is identical to those of the grasper, scalpel, and needle-holder described above. Jaws 191 and 193 are actuated by a toggle clamp mechanism including two lever arms 192 and 194, and four pivot points 195a to 195d. A small downward force exerted by a surgeon's thumb in the location of pivot pin 195b is amplified through the toggle clamp mechanism into a large force at pivot point 195c. The bent arm configuration of jaw 191 translates the large force at pivot point 195c into a very large clamping force between jaws 191 and 193.

When lever arms 192 and 194 are in a straight line relationship, the needle holder is in an essentially locked position. An upward force exerted on pivot point 195c, for example, by a surgeon's fingers, or by a separate release lever (not shown), moves the lever arms 192 and 194 into an open position, releasing the clamping force between the jaws.

In another embodiment, the pivot points are replaced by so-called "living" hinges, and the lever arms 192 and 194, and bent arm jaw 191, as well as lower jaw 193 and lower arm 197, can all be made of a single piece of plastic or other material having the living hinges in place of each pivot point 195a to 195d.

### Electrocauterizing Instruments

Any of the surgical instruments described above, when made of an electrically conducting material, can be adapted to provide an electrocautery feature.

For example, any tool including two jaws can be easily adapted to provide bipolar electrocautery. Such an adaptation requires that the harness or mount be made of an insulating material such as plastic, or be electrically insulated from the retracting/deploying mechanism or tool, e.g., coated with a standard electrosurgical insulation. Further, the retracting/deploying mechanism and the tool must be insulated as well, except for the cauterizing tip of the tool.

As an alternative, only one of the jaws of the tool could be used for monopolar electrocautery, and the other jaw could be made of an insulating material such as plastic. The plastic coating would cover the entire length of the instrument with a very small bare portion at the most distal tip of the instrument. Because the instruments of the invention are mounted directly on the surgeon's fingertips, the bipolar cautery embodiment is the preferred method of electrocautery.

To adapt a particular instrument, e.g., the grasper described above, for bipolar electrocautery, moving component 40 and moving jaw 41 are electrically insulated from retracting/deploying mechanism 30 and stationary jaw 31 with ceramic disks or wafers around pivot pin 43 (the pivot pin is preferably coated with an insulating material, e.g., ceramic), and harness 20 also is made of a plastic or ceramic material to insulate the surgeon. Furthermore, attachment stud 50 is preferably made of, or coated with, an insulating material, e.g., a ceramic. Electrically conductive fittings are attached, e.g., soldered or machined in place, on both the moving component 40 and the retracting/deploying mechanism 30. Wires are connected to these fittings at one of their ends, and are connected at their other ends to a controllable power source.

During bipolar operation, current flows from one jaw, e.g., moving jaw 41, to the other jaw, e.g., stationary jaw 31, through the target tissue to be cauterized between the two jaws. During monopolar operation, current flows from one scalpel, or other instrument, into the target tissue, and through the patient to a ground. In both embodiments, the majority of the surfaces of the one or two jaws are preferably insulated so that the current flow can be controlled.

### Fluid Conduits

Any of the instruments described above can be outfitted with fluid conduits for both irrigation and aspiration using standard components and methods. Commercially available fluid tubing sets can be connected to the instruments, e.g., to the tool harness or mount, and run along the surgeons finger and arm, and attached to standard pumping mechanisms (such as those made by Smith & Nephew Dyonics, MA and Davol, RI). Aspiration conduits are connected to standard vacuum ports.

Control buttons for actuating and stopping irrigation and suction can be located on flexible appendages attached to the proximal end of the harness and arranged on or along the surgeon's finger or fingers so that the surgeon can press the buttons with the thumb. Alternatively, these functions could be controlled by a foot pedal or switch that controls the flow of fluids or vacuum. These foot pedals come as standard equipment with most fluid and vacuum pumps.

### Manufacture of Fingertip-Mounted

### Minimally Invasive Surgical Instruments

The surgical instruments can be constructed using known manufacturing techniques and materials. For example, the mount, e.g., harness, can be injection molded, and can be the same size and shape in different types of instruments; thus the harness is the most likely candidate for mass production. Similarly, the finger mounts can also be mass-produced, e.g., extruded from SANTOPRENE^{®}, or stamped or cut from laminated sheets of flexible fabrics, rubbers, or other materials, as described above.

For reusable instruments, the stationary and moving jaws of the tools, and the retracting/deploying mechanism, are preferably machined from a surgical stainless steel or other metal using standard machine practices. As an example, the gripping surfaces and fenestrations described for the grasper can be created by electron discharge machining (EDM). In particular, the teeth of the gripping surfaces can be created by wire EDM, and the fenestrations can be made using ram or sinker EDM. The cutouts and cavities, e.g., for the spring, can be created by similar EDM procedures.

Instruments with functional tips such as scalpels or scissors that include blades or edges that must be kept extremely sharp, as well as tools used for electrocautery, where the degree of burning or charring would make the instruments difficult to clean, are preferably produced as disposable instruments. These disposable instruments are constructed of plastics with metal inserts molded or ultrasonically adhered in place. Instruments that are intended to be used as graspers and soft tissue dissectors can be made to be reusable and sterilizable. The sterilizable instruments are preferably constructed of all stainless steel, or of autoclavable plastic with stainless steel tool tips. Alternatively, both the disposable and reusable instruments can be manufactured using metal-injection-molding techniques.

### Methods of Use

The fingertip-mounted instruments are best used in hand-assisted minimally invasive surgical procedures within the abdomen or pelvis, such as are currently performed by laparoscopic surgery. Other sites within the body can be accessed as well. In addition, the new instruments can be used in traditional, open surgery as well, in place of traditional surgical instruments such as forceps, scalpels, and scissors.

In a minimally invasive setting, after the body cavity is insufflated (with a standard insufflator), the surgeon performs a visual diagnosis of the inside of the cavity using standard laparoscopic techniques through a small incision. A standard laparoscope or other device is used throughout the surgery to enable constant visualization of the interior of the cavity. Once an accurate diagnosis is made, the surgeon locates and creates a new incision of about 5.08 to 7.62 cm (2 to 3 inches), depending on the size of his or her hand and wrist, prepares a standard hand access port, and palpates the inside the patient cavity. The hand access port can be, e.g., an INTROMIT^{™} (Medtech, Dublin, Ireland) port that enables the surgeon to introduce a hand into the cavity and remove it without loss of peritoneum. The Pneumo-Dexterity Sleeve^{™} (Pilling Weck, N.C.) can also be used as the hand access port.

The surgeon then mounts an instrument on a gloved finger, and can mount additional instruments onto other fingers including the thumb. As an alternative, one or more mounts can be permanently secured, e.g., by adhesive, metal, or plastic staples, or other means, on the fingertips of a sterilized glove, e.g., of a cloth mesh or weave material. Initially, the instruments are attached to the mounts in the retracted position (if they feature a retracted position).

In the case of the tool/finger mount systems, the finger mounts, e.g., finger-sleeves, are mounted empty (or with a tool already attached), and tools can be interchangeably attached and disconnected, e.g., from the pocket of the sleeve. A surgeon can test the position of the mount by inserting a tool into the sleeve pocket, and actuating the tool to see whether the tool functions in its intended fashion, and then fixing the sleeve's position, e.g., with surgical tape.

Once the patient cavity is ready for surgery, the surgeon inserts his or her hand equipped with the instrument or instruments through the hand access port. The surgeon then performs the required resection, dissection, ligation, etc. by instrument. When the need arises for, e.g., blunt dissection, running bowels, or probing for tumors, the surgeon either retracts the tool (if retractable) or removes the hand from the patient, and then either disconnects the tool from the mount or removes the entire mount from his or her finger. The surgeon then makes full use of his or her unencumbered hand to manipulating tissues, etc. inside the body cavity.

## Claims

1. A finger mount for use by a surgeon comprising a finger contacting component (20,90,100,142,152,160) having an inner and an outer surface with the inner surface contoured and dimensioned to fit onto a finger, and an attachment mechanism (50,68,88,98,108,145,164) connected to the outer surface of the finger contacting component,
**CHARACTERIZED IN THAT**
the attachment mechanism is rotatable relative to the finger contacting component, and configured to connect to an interchangeable miniature surgical instrument (30,40,162,180), and to enable the surgeon to actuate or deploy the instrument by use of another digit to effect rotation of the surgical instrument and thereby of said attachment mechanism with respect to the finger contacting component to actuate/deploy the surgical instrument.

2. A finger mount according to Claim 1 wherein the finger contacting component is in the form of a semi-circle that contacts only a portion of a finger.

3. A finger mount according to Claim 1 wherein the finger contacting component forms a cylinder that encircles the finger in use.

4. A finger mount according to any preceding Claim wherein the attachment mechanism is a protruding member (50) that connects to a corresponding recess in the miniature surgical tool.

5. A finger mount according to any of Claims 1 to 4 wherein the attachment mechanism is a recess (145,155) that connects to a corresponding protruding member (143,153) in the miniature surgical tool.

6. A finger mount according to any preceding Claim wherein the finger contacting component is configured to fit onto a thumb.

7. A finger mount according to any of Claims 1 to 5 wherein the finger contacting component comprises one or more straps to secure the component to the finger.

8. A finger mount according to Claim 7 wherein the one or more straps each comprise a malleable wire that can be wrapped around the finger to secure the component.

9. A finger mount according to Claim 7 wherein the one or more straps are one or more wings (92), and the wing or wings are secured to themselves or each other by mechanical snaps (93,94).

10. A finger mount according to Claim 7 wherein the one or more straps are one or more wings (102), and the wing or wings are secured to themselves or each other by an adhesive.

11. A finger mount according to any of Claims 1 to 5 wherein the finger contacting component (160) is secured to the finger by an adhesive on the inner surface of the component.

12. A minimally invasive surgical system comprising a finger mount according to any preceding Claim, and a miniature surgical tool including a connector configured to connect to the attachment mechanism of the finger mount.

13. A system according to Claim 12 wherein the miniature surgical tool is one of a grasper, a scalpel, a needle holder, a scissor, a camera, and an electrocautery probe.

14. A system according to Claim 12 or Claim 13 wherein the miniature surgical tool is permanently attached to the finger mount.

## Patentansprüche

1. Fingerhalter für den Gebrauch durch einen Chirurgen, der Folgendes umfasst: einen Fingerkontaktteil (20, 90, 100, 142, 152, 160) mit einer Innen- und einer Außenfläche, wobei die Innenfläche so konturiert und dimensioniert ist, dass sie auf einen Finger passt, und einen Befestigungsmechanismus (50, 68, 88, 98, 108, 145, 164), der mit der Außenfläche des Fingerkontaktteils verbunden ist,
**dadurch gekennzeichnet, dass**
der Befestigungsmechanismus relativ zu dem Fingerkontaktteil drehbar und so konfiguriert ist, dass er mit einem austauschbaren chirurgischen Miniaturinstrument (30, 40, 162, 180) verbunden werden kann und den Chirurgen befähigt, das Instrument mit einem anderen Finger zu betätigen oder einzusetzen, um eine Rotation des chirurgischen Instruments und **dadurch** des genannten Befestigungsmechanismus mit Bezug auf den Fingerkontaktteil zu bewirken, um das chirurgische Instrument zu betätigen/einzusetzen.

2. Fingerhalter nach Anspruch 1, wobei der Fingerkontaktteil die Form eines Halbkreises hat, der nur mit einem Teil eines Fingers in Kontakt ist.

3. Fingerhalter nach Anspruch 1, wobei der Fingerkontaktteil die Form eines Zylinders hat, der den Finger beim Gebrauch umgibt.

4. Fingerhalter nach einem der vorherigen Ansprüche, wobei der Befestigungsmechanismus ein vorstehendes Element (50) ist, das mit einer entsprechenden Aussparung in dem chirurgischen Minitaturwerkzeug verbunden werden kann.

5. Fingerhalter nach einem der Ansprüche 1 bis 4, wobei der Befestigungsmechanismus eine Aussparung (145, 155) ist, die mit einem entsprechenden vorstehenden Element (143, 153) in dem chirurgischen Werkzeug verbunden werden kann.

6. Fingerhalter nach einem der vorherigen Ansprüche, wobei der Fingerkontaktteil so konfiguriert ist, dass er auf einen Daumen passt.

7. Fingerhalter nach einem der Ansprüche 1 bis 5, wobei der Fingerkontaktteil einen oder mehrere Riemen zum Befestigen des Teils am Finger aufweist.

8. Fingerhalter nach Anspruch 7, wobei der/die eine oder mehreren Riemen jeweils einen unter Druck verformbaren Draht aufweist/aufweisen, der zum Befestigen des Teils um den Finger gewickelt werden kann.

9. Fingerhalter nach Anspruch 7, wobei der/die eine oder mehreren Riemen ein oder mehrere Flügel (92) ist/sind und der/die Flügel durch mechanische Verschlüsse (93, 94) an sich oder aneinander befestigt werden.

10. Fingerhalter nach Anspruch 7, wobei der/die eine oder mehreren Riemen ein oder mehrere Flügel (92) ist/sind und der/die Flügel mit einem Klebstoff an sich oder aneinander befestigt werden.

11. Fingerhalter nach einem der Ansprüche 1 bis 5, wobei der Fingerkontaktteil (160) mit einem Klebstoff an der Innenfläche des Teils am Finger befestigt wird.

12. Minimal invasives chirurgisches System, das einen Fingerhalter nach einem der vorherigen Ansprüche und ein chirurgisches Miniaturwerkzeug mit einem Verbinder umfasst, der so konfiguriert ist, dass er mit dem Befestigungsmechanismus des Fingerhalters verbunden werden kann.

13. System nach Anspruch 12, wobei das chirurgische Miniaturwerkzeug ein Greifer, ein Skalpell, ein Nadelhalter, eine Schere, eine Kamera oder eine Elektrokauter-Sonde ist.

14. System nach Anspruch 12 oder Anspruch 13, wobei das chirurgische Miniaturwerkzeug permanent an dem Fingerhalter angebracht ist.

## Revendications

1. Dispositif monté sur doigt conçu pour être utilisé par un chirurgien, ledit dispositif comprenant un composant de contact avec un doigt (20, 90, 100, 142, 152, 160) qui présente une surface interne et une surface externe et dont le contour et les dimensions de la surface interne sont adaptés à un doigt, et un mécanisme d'ancrage (50, 68, 88, 98, 108, 145, 164) raccordé à la surface externe du composant de contact avec un doigt,
**CARACTÉRISÉ EN CE QUE**
le mécanisme d'ancrage est orientable par rapport au composant de contact avec un doigt et configuré de façon à être couplé à un instrument chirurgical miniature interchangeable (30, 40, 162, 180) et à permettre au chirurgien d'actionner ou de déployer l'instrument en utilisant un autre doigt pour produire une rotation de l'instrument chirurgical et par là même dudit mécanisme d'ancrage par rapport au composant de contact avec un doigt afin d'actionner/de déployer l'instrument chirurgical.

2. Dispositif monté sur doigt selon la revendication 1, où le composant de contact avec un doigt a une forme semi-circulaire qui assure un contact avec une portion d'un doigt seulement.

3. Dispositif monté sur doigt selon la revendication 1, où le composant de contact avec un doigt forme un cylindre qui encercle le doigt durant l'utilisation.

4. Dispositif monté sur doigt selon l'une quelconque des revendications précédentes, où le mécanisme d'ancrage est une barre en saillie (50) qui est embrochée dans un évidement correspondant de l'outil chirurgical miniature.

5. Dispositif monté sur doigt selon l'une quelconque des revendications 1 à 4, où le mécanisme d'ancrage est un évidement (145, 155) dans lequel est embrochée une barre en saillie correspondante (143, 153) de l'outil chirurgical miniature.

6. Dispositif monté sur doigt selon l'une quelconque des revendications précédentes, où le composant de contact avec un doigt est configuré pour s'adapter à un pouce.

7. Dispositif monté sur doigt selon l'une quelconque des revendications 1 à 5, où le composant de contact avec un doigt comprend une ou plusieurs brides de fixation permettant d'arrimer solidement le composant sur le doigt.

8. Dispositif monté sur doigt selon la revendication 7, où la ou les brides de fixation comportent chacune un fil métallique malléable qui peut être enveloppé autour du doigt pour immobiliser le composant.

9. Dispositif monté sur doigt selon la revendication 7, où la ou les brides de fixation correspondent à une ou plusieurs ailettes (92), l'ailette ou les ailettes étant rattachée(s) à elle(s)-même(s) ou les unes aux autres au moyen de cliquets mécaniques (93, 94).

10. Dispositif monté sur doigt selon la revendication 7, où la ou les brides de fixation correspondent à une ou plusieurs ailettes (102), l'ailette ou les ailettes étant rattachée(s) à elle(s)-même(s) ou les unes aux autres au moyen d'une lamelle adhésive.

11. Dispositif monté sur doigt selon l'une quelconque des revendications 1 à 5, où le composant de contact avec un doigt (160) est arrimé au doigt au moyen d'une lamelle adhésive située sur la surface interne du composant.

12. Système chirurgical au minimum invasif qui comprend un dispositif monté sur doigt selon l'une quelconque des revendications précédentes et un outil chirurgical miniature comportant un connecteur configuré pour s'embrocher dans le mécanisme d'ancrage du dispositif monté sur doigt.

13. Système selon la revendication 12, où l'outil chirurgical miniature est l'un des suivants : pince de saisie, scalpel, porte-aiguille, ciseau, caméra et cathéter d'électrocautérisation.

14. Système selon la revendication 12 ou la revendication 13, où l'outil chirurgical miniature est en permanence arrimé sur le dispositif monté sur doigt.
